# EUROPEAN PATENT APPLICATION

(11) **EP 1 669 088 A1**
(43) Date of publication of application: **14.06.2006**
(21) Application number: 04788405.1
(22) Date of filing: 30.09.2004
(51) Int. Cl.: A61K 45/00, A61P 3/10, C12N 1/38, C12N 15/57

(54) **METHOD OF TREATING DIABETES BY INHIBTING DEGRADATION OF AT LEAST ONE OF CREBL1, ATF6 and HNF-4 ALPHA BY HtrA2**

(30) Priority: 30.09.2003 JP 2003342587
(71) Applicant: DAIICHI PHARMACEUTICAL CO., LTD., Chuo-ku, Tokyo 103-8234 (JP)
(72) Inventor: DOI, Hirofumi, c/o Celestar Lexico-Sciences, Inc., Mihama-ku, Chiba-shi, Chiba 2618501 (JP); SAITO, Ken, c/o Daiichi Pharmaceutical Co., Ltd., Edogawa-ku, Tokyo 1348630 (JP)
(74) Representative: De Clercq, Ann G. Y.
(86) International application number: PCT/JP2004/014377
(87) International publication number: WO 2005/030255

(57) **Abstract**

The present invention found the interaction of CREBL1 and HNF-4α with HtrA2 and revealed for the first time that CREBL1, ATF6, and HNF-4α are degraded by active HtrA2.

In addition, the present invention provides a means for inhibiting the degradation of at least one of CREBL1, ATF6, and HNF-4α, comprising inhibiting the function of HtrA2; a means for preventing and/or treating diabetes, comprising inhibiting the degradation by HtrA2 of at least one of CREBL1, ATF6, and HNF-4α; a means for preventing cell death (for example, pancreatic *β* cell death), comprising inhibiting the degradation by HtrA2 of CREBL1 and/or ATF6; a means for preventing and/or treating type 2 diabetes, comprising inhibiting the degradation by HtrA2 of HNF-4α and a reagent kit.

SEQUENCE LISTING

<110>  DAIICHI PHARMACEUTICAL CO., LTD.

<120>  Method for treating diabetes by inhibiting degradation of at
       least one of CREBL1, ATF6 and HNF-4a by HtrA2

<130>  DAII-001-EP

<140>  04788405.1
<141>  2004-09-30

<150>  JP P2003-342587
<151>  2003-09-30

<160>  35

<170>  PatentIn version 3.3

<210>  1
<211>  1377
<212>  DNA
<213>  Homo sapiens


<220>
<221>  misc_feature
<223>  DNA that codes for HtrA2 precursor protein

<400>  1
atggctgcgc cgagggcggg gcggggtgca ggctggagcc ttcgggcatg gcgggctttg      60

gggggcattc gctgggggag gagaccccgt ttgacccctg acctccgggc cctgctgacg     120

tcaggaactt ctgacccccg ggcccgagtg acttatggga cccccagtct ctgggcccgg     180

ttgtctgttg gggtcactga accccgagca tgcctgacgt ctgggacccc gggtccccgg     240

gcacaactga ctgcggtgac cccagatacc aggacccggg aggcctcaga gaactctgga     300

acccgttcgc gcgcgtggct ggcggtggcg ctgggcgctg ggggggcagt gctgttgttg     360

ttgtggggcg ggggtcgggg tcctccggcc gtcctcgccg ccgtccctag cccgccgccc     420

gcttctcccc ggagtcagta caacttcatc gcagatgtgg tggagaagac agcacctgcc     480

gtggtctata tcgagatcct ggaccggcac cctttcttgg gccgcgaggt ccctatctcg     540

aacggctcag gattcgtggt ggctgccgat gggctcattg tcaccaacgc ccatgtggtg     600

gctgatcggc gcagagtccg tgtgagactg ctaagcggcg acacgtatga ggccgtggtc     660

acagctgtgg atcccgtggc agacatcgca acgctgagga ttcagactaa ggagcctctc     720

cccacgctgc ctctgggacg ctcagctgat gtccggcaag gggagtttgt tgttgccatg     780

ggaagtccct ttgcactgca gaacacgatc acatccggca ttgttagctc tgctcagcgt     840

ccagccagag acctgggact cccccaaacc aatgtggaat acattcaaac tgatgcagct     900

attgattttg gaaactctgg aggtcccctg gttaacctgg atggggaggt gattggagtg     960

aacaccatga aggtcacagc tggaatctcc tttgccatcc cttctgatcg tcttcgagag    1020

tttctgcatc gtggggaaaa gaagaattcc tcctccggaa tcagtgggtc ccagcggcgc    1080

tacattgggg tgatgatgct gaccctgagt cccagcatcc ttgctgaact acagcttcga    1140

gaaccaagct ttcccgatgt tcagcatggt gtactcatcc ataaagtcat cctgggctcc    1200

cctgcacacc gggctggtct gcggcctggt gatgtgattt tggccattgg ggagcagatg    1260

gtacaaaatg ctgaagatgt ttatgaagct gttcgaaccc aatcccagtt ggcagtgcag    1320

atccggcggg gacgagaaac actgacctta tatgtgaccc ctgaggtcac agaatga       1377

<210>  2
<211>  458
<212>  PRT
<213>  Homo sapiens

<220>
<221>  misc_feature
<223>  HtrA2 precursor protein

<400>  2

<210>  3
<211>  981
<212>  DNA
<213>  Homo sapiens

<220>
<221>  misc_feature
<223>  DNA that codes for mature HtrA2

<400>  3
atggccgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaact ctggaggtcc cctggttaac     540

ctggatgggg aggtgattgg agtgaacacc atgaaggtca cagctggaat ctcctttgcc     600

atcccttctg atcgtcttcg agagtttctg catcgtgggg aaaagaagaa ttcctcctcc     660

ggaatcagtg ggtcccagcg gcgctacatt ggggtgatga tgctgaccct gagtcccagc     720

atccttgctg aactacagct tcgagaacca agctttcccg atgttcagca tggtgtactc     780

atccataaag tcatcctggg ctcccctgca caccgggctg gtctgcggcc tggtgatgtg     840

attttggcca ttggggagca gatggtacaa aatgctgaag atgtttatga agctgttcga     900

acccaatccc agttggcagt gcagatccgg cggggacgag aaacactgac cttatatgtg     960

acccctgagg tcacagaatg a                                               981

<210>  4
<211>  326
<212>  PRT
<213>  Homo sapiens

<220>
<221>  misc_feature
<223>  mature HtrA2

<400>  4

Met Ala Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser

35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ser Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val

305                 310                 315                 320

Thr Pro Glu Val Thr Glu
                325

<210>  5
<211>  981
<212>  DNA
<213>  Artificial

<220>
<223>  Polynucleotide consisting of the same base sequence of SEQ ID
       NO:3 wherein the nucleotide of position 520 is g

<220>
<221>  misc_feature
<223>  DNA that codes for mature HtrA2(S306A)

<400>  5
atggccgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaacg ctggaggtcc cctggttaac     540

ctggatgggg aggtgattgg agtgaacacc atgaaggtca cagctggaat ctcctttgcc     600

atcccttctg atcgtcttcg agagtttctg catcgtgggg aaaagaagaa ttcctcctcc     660

ggaatcagtg ggtcccagcg gcgctacatt ggggtgatga tgctgaccct gagtcccagc     720

atccttgctg aactacagct tcgagaacca agctttcccg atgttcagca tggtgtactc     780

atccataaag tcatcctggg ctcccctgca caccgggctg gtctgcggcc tggtgatgtg     840

attttggcca ttggggagca gatggtacaa aatgctgaag atgtttatga agctgttcga     900

acccaatccc agttggcagt gcagatccgg cggggacgag aaacactgac cttatatgtg     960

acccctgagg tcacagaatg a                                               981

<210>  6
<211>  326
<212>  PRT
<213>  Artificial

<220>
<223>  Polypeptide consisting of the same amino acid sequence of SEQ ID
       NO:4 wherein the 174th amino acid residue is substituted by Ala

<220>

<221>  misc_feature
<223>  mature HtrA2(S306A)

<400>  6

Met Ala Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ala Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
        260                 265                 270

Ala Gly Leu Arg Pro Gly Asp Val Ile Leu Ala Ile Gly Glu Gln Met
    275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  7
<211>  969
<212>  DNA
<213>  Artificial

<220>
<223>  Polynucleotide consisting of the same base sequence of SEQ ID
       NO:3 wherein the nucleotides of position 4-15 are deleted

<220>
<221>  misc_feature
<223>  DNA that codes for mature HtrA2(delta AVPS)

<400>  7
atgccgccgc ccgcttctcc ccggagtcag tacaacttca tcgcagatgt ggtggagaag      60

acagcacctg ccgtggtcta tatcgagatc ctggaccggc accctttctt gggccgcgag     120

gtccctatct cgaacggctc aggattcgtg gtggctgccg atgggctcat tgtcaccaac     180

gcccatgtgg tggctgatcg gcgcagagtc cgtgtgagac tgctaagcgg cgacacgtat     240

gaggccgtgg tcacagctgt ggatcccgtg gcagacatcg caacgctgag gattcagact     300

aaggagcctc tccccacgct gcctctggga cgctcagctg atgtccggca aggggagttt     360

gttgttgcca tgggaagtcc ctttgcactg cagaacacga tcacatccgg cattgttagc     420

tctgctcagc gtccagccag agacctggga ctcccccaaa ccaatgtgga atacattcaa     480

actgatgcag ctattgattt tggaaactct ggaggtcccc tggttaacct ggatggggag     540

gtgattggag tgaacaccat gaaggtcaca gctggaatct cctttgccat cccttctgat     600

cgtcttcgag agtttctgca tcgtggggaa aagaagaatt cctcctccgg aatcagtggg     660

tcccagcggc gctacattgg ggtgatgatg ctgaccctga gtcccagcat ccttgctgaa     720

ctacagcttc gagaaccaag ctttcccgat gttcagcatg gtgtactcat ccataaagtc     780

atcctgggct cccctgcaca ccgggctggt ctgcggcctg gtgatgtgat tttggccatt     840

ggggagcaga tggtacaaaa tgctgaagat gtttatgaag ctgttcgaac ccaatcccag     900

ttggcagtgc agatccggcg gggacgagaa acactgacct tatatgtgac ccctgaggtc     960

acagaatga                                                          969

<210>  8
<211>  322
<212>  PRT
<213>  Artificial

<220>
<223>  Polypeptide consisting of the same amino acid sequence of SEQ ID
       NO:4 wherein the amino acid residues from the 2nd to the 5th are
       deleted

<220>
<221>  misc_feature
<223>  mature HtrA2(delta AVPS)

<400>  8

Met Pro Pro Pro Ala Ser Pro Arg Ser Gln Tyr Asn Phe Ile Ala Asp
1               5                   10                  15

Val Val Glu Lys Thr Ala Pro Ala Val Val Tyr Ile Glu Ile Leu Asp
            20                  25                  30

Arg His Pro Phe Leu Gly Arg Glu Val Pro Ile Ser Asn Gly Ser Gly
        35                  40                  45

Phe Val Val Ala Ala Asp Gly Leu Ile Val Thr Asn Ala His Val Val
    50                  55                  60

Ala Asp Arg Arg Arg Val Arg Val Arg Leu Leu Ser Gly Asp Thr Tyr
65                  70                  75                  80

Glu Ala Val Val Thr Ala Val Asp Pro Val Ala Asp Ile Ala Thr Leu
                85                  90                  95

Arg Ile Gln Thr Lys Glu Pro Leu Pro Thr Leu Pro Leu Gly Arg Ser
            100                 105                 110

Ala Asp Val Arg Gln Gly Glu Phe Val Val Ala Met Gly Ser Pro Phe
        115                 120                 125

Ala Leu Gln Asn Thr Ile Thr Ser Gly Ile Val Ser Ser Ala Gln Arg
    130                 135                 140

Pro Ala Arg Asp Leu Gly Leu Pro Gln Thr Asn Val Glu Tyr Ile Gln
145                 150                 155                 160

Thr Asp Ala Ala Ile Asp Phe Gly Asn Ser Gly Gly Pro Leu Val Asn
                165                 170                 175

Leu Asp Gly Glu Val Ile Gly Val Asn Thr Met Lys Val Thr Ala Gly
            180                 185                 190

Ile Ser Phe Ala Ile Pro Ser Asp Arg Leu Arg Glu Phe Leu His Arg
        195                 200                 205

Gly Glu Lys Lys Asn Ser Ser Ser Gly Ile Ser Gly Ser Gln Arg Arg
    210                 215                 220

Tyr Ile Gly Val Met Met Leu Thr Leu Ser Pro Ser Ile Leu Ala Glu
225                 230                 235                 240

Leu Gln Leu Arg Glu Pro Ser Phe Pro Asp Val Gln His Gly Val Leu
                245                 250                 255

Ile His Lys Val Ile Leu Gly Ser Pro Ala His Arg Ala Gly Leu Arg
            260                 265                 270

Pro Gly Asp Val Ile Leu Ala Ile Gly Glu Gln Met Val Gln Asn Ala
        275                 280                 285

Glu Asp Val Tyr Glu Ala Val Arg Thr Gln Ser Gln Leu Ala Val Gln
    290                 295                 300

Ile Arg Arg Gly Arg Glu Thr Leu Thr Leu Tyr Val Thr Pro Glu Val
305                 310                 315                 320

Thr Glu

<210>  9
<211>  981
<212>  DNA
<213>  Artificial

<220>
<223>  Polynucleotide consisting of the same base sequence of SEQ ID
       NO:3 wherein the nucleotide of position 5 is g

<220>
<221>  misc_feature
<223>  DNA that codes for mature HtrA2(GVPS)

<400>  9
atgggcgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaact ctggaggtcc cctggttaac     540

ctggatgggg aggtgattgg agtgaacacc atgaaggtca cagctggaat ctcctttgcc 600

atcccttctg atcgtcttcg agagtttctg catcgtgggg aaaagaagaa ttcctcctcc 660

ggaatcagtg ggtcccagcg gcgctacatt ggggtgatga tgctgaccct gagtcccagc 720

atccttgctg aactacagct tcgagaacca agctttcccg atgttcagca tggtgtactc 780

atccataaag tcatcctggg ctcccctgca caccgggctg gtctgcggcc tggtgatgtg 840

attttggcca ttggggagca gatggtacaa aatgctgaag atgtttatga agctgttcga 900

acccaatccc agttggcagt gcagatccgg cggggacgag aaacactgac cttatatgtg 960

acccctgagg tcacagaatg a 981

<210> 10
<211> 326
<212> PRT
<213> Artificial

<220>
<223> Polypeptide consisting of the same amino acid sequence of SEQ ID
NO:4 wherein the 2nd amino acid residue is substituted by Gly

<220>
<221> misc_feature
<223> mature HtrA2(GVPS)

<400> 10

Met Gly Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile
            20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ser Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln
    290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  11
<211>  969
<212>  DNA
<213>  Artificial

<220>
<223>  Polynucleotide consisting of the same base sequence of SEQ ID
       NO:5 wherein the nucleotides of position 4-15 are deleted

<220>
<221>  misc_feature
<223>  DNA that codes for mature HtrA2(S306A, delta AVPS)

<400>  11
atgccgccgc ccgcttctcc ccggagtcag tacaacttca tcgcagatgt ggtggagaag      60

acagcacctg ccgtggtcta tatcgagatc ctggaccggc accctttctt gggccgcgag     120

gtccctatct cgaacggctc aggattcgtg gtggctgccg atgggctcat tgtcaccaac     180

gcccatgtgg tggctgatcg gcgcagagtc cgtgtgagac tgctaagcgg cgacacgtat     240

gaggccgtgg tcacagctgt ggatcccgtg gcagacatcg caacgctgag gattcagact     300

aaggagcctc tccccacgct gcctctggga cgctcagctg atgtccggca aggggagttt     360

gttgttgcca tgggaagtcc ctttgcactg cagaacacga tcacatccgg cattgttagc     420

tctgctcagc gtccagccag agacctggga ctcccccaaa ccaatgtgga atacattcaa     480

actgatgcag ctattgattt tggaaacgct ggaggtcccc tggttaacct ggatggggag     540

gtgattggag tgaacaccat gaaggtcaca gctggaatct cctttgccat cccttctgat     600

cgtcttcgag agtttctgca tcgtggggaa aagaagaatt cctcctccgg aatcagtggg     660

tcccagcggc gctacattgg ggtgatgatg ctgaccctga gtcccagcat ccttgctgaa     720

ctacagcttc gagaaccaag ctttcccgat gttcagcatg gtgtactcat ccataaagtc     780

atcctgggct cccctgcaca ccgggctggt ctgcggcctg gtgatgtgat tttggccatt     840

ggggagcaga tggtacaaaa tgctgaagat gtttatgaag ctgttcgaac ccaatcccag     900

ttggcagtgc agatccggcg gggacgagaa acactgacct tatatgtgac ccctgaggtc     960

acagaatga                                                             969

<210>  12
<211>  322
<212>  PRT
<213>  Artificial

<220>
<223>  Polypeptide consisting of the same amino acid sequence of SEQ ID
       NO:6 wherein the amino acid residues from the 2nd to the 5th are
       deleted

<220>
<221>  misc_feature
<223>  mature HtrA2(S306A, delta AVPS)

<400>  12

Met Pro Pro Pro Ala Ser Pro Arg Ser Gln Tyr Asn Phe Ile Ala Asp
1               5                   10                  15

Val Val Glu Lys Thr Ala Pro Ala Val Val Tyr Ile Glu Ile Leu Asp
            20                  25                  30

Arg His Pro Phe Leu Gly Arg Glu Val Pro Ile Ser Asn Gly Ser Gly
        35                  40                  45

Phe Val Val Ala Ala Asp Gly Leu Ile Val Thr Asn Ala His Val Val
    50                  55                  60

Ala Asp Arg Arg Arg Val Arg Val Arg Leu Leu Ser Gly Asp Thr Tyr
65                  70                  75                  80

Glu Ala Val Val Thr Ala Val Asp Pro Val Ala Asp Ile Ala Thr Leu
                85                  90                  95

Arg Ile Gln Thr Lys Glu Pro Leu Pro Thr Leu Pro Leu Gly Arg Ser
            100                 105                 110

Ala Asp Val Arg Gln Gly Glu Phe Val Val Ala Met Gly Ser Pro Phe
        115                 120                 125

Ala Leu Gln Asn Thr Ile Thr Ser Gly Ile Val Ser Ser Ala Gln Arg
    130                 135                 140

Pro Ala Arg Asp Leu Gly Leu Pro Gln Thr Asn Val Glu Tyr Ile Gln
145                 150                 155                 160

Thr Asp Ala Ala Ile Asp Phe Gly Asn Ala Gly Gly Pro Leu Val Asn
                165                 170                 175

Leu Asp Gly Glu Val Ile Gly Val Asn Thr Met Lys Val Thr Ala Gly
            180                 185                 190

Ile Ser Phe Ala Ile Pro Ser Asp Arg Leu Arg Glu Phe Leu His Arg
        195                 200                 205

Gly Glu Lys Lys Asn Ser Ser Ser Gly Ile Ser Gly Ser Gln Arg Arg
    210                 215                 220

Tyr Ile Gly Val Met Met Leu Thr Leu Ser Pro Ser Ile Leu Ala Glu
225                 230                 235                 240

Leu Gln Leu Arg Glu Pro Ser Phe Pro Asp Val Gln His Gly Val Leu
                245                 250                 255

Ile His Lys Val Ile Leu Gly Ser Pro Ala His Arg Ala Gly Leu Arg
            260                 265                 270

Pro Gly Asp Val Ile Leu Ala Ile Gly Glu Gln Met Val Gln Asn Ala
        275                 280                 285

Glu Asp Val Tyr Glu Ala Val Arg Thr Gln Ser Gln Leu Ala Val Gln
    290                 295                 300

Ile Arg Arg Gly Arg Glu Thr Leu Thr Leu Tyr Val Thr Pro Glu Val
305                 310                 315                 320

Thr Glu


<210>  13
<211>  981
<212>  DNA

<213>  Artificial

<220>
<223>  Polynucleotide consisting of the same base sequence of SEQ ID
      NO:5 wherein the nucleotide of position 5 is g

<220>
<221>  misc_feature
<223>  DNA that codes for mature HtrA2(S306A, GVPS)

<400>  13
atgggcgtcc ctagcccgcc gcccgcttct ccccggagtc agtacaactt catcgcagat      60

gtggtggaga agacagcacc tgccgtggtc tatatcgaga tcctggaccg gcaccctttc     120

ttgggccgcg aggtccctat ctcgaacggc tcaggattcg tggtggctgc cgatgggctc     180

attgtcacca acgcccatgt ggtggctgat cggcgcagag tccgtgtgag actgctaagc     240

ggcgacacgt atgaggccgt ggtcacagct gtggatcccg tggcagacat cgcaacgctg     300

aggattcaga ctaaggagcc tctccccacg ctgcctctgg gacgctcagc tgatgtccgg     360

caaggggagt ttgttgttgc catgggaagt ccctttgcac tgcagaacac gatcacatcc     420

ggcattgtta gctctgctca gcgtccagcc agagacctgg gactccccca aaccaatgtg     480

gaatacattc aaactgatgc agctattgat tttggaaacg ctggaggtcc cctggttaac     540

ctggatgggg aggtgattgg agtgaacacc atgaaggtca cagctggaat ctcctttgcc     600

atcccttctg atcgtcttcg agagtttctg catcgtgggg aaaagaagaa ttcctcctcc     660

ggaatcagtg ggtcccagcg gcgctacatt ggggtgatga tgctgaccct gagtcccagc     720

atccttgctg aactacagct tcgagaacca agctttcccg atgttcagca tggtgtactc     780

atccataaag tcatcctggg ctcccctgca caccgggctg gtctgcggcc tggtgatgtg     840

attttggcca ttggggagca gatggtacaa aatgctgaag atgtttatga agctgttcga     900

acccaatccc agttggcagt gcagatccgg cggggacgag aaacactgac cttatatgtg     960

acccctgagg tcacagaatg a                                               981

<210>  14
<211>  326
<212>  PRT
<213>  Artificial

<220>
<223>  Polypeptide consisting of the same amino acid sequence of SEQ ID
       NO:6 wherein the 2nd amino acid residue is substituted by Gly

<220>
<221>  misc_feature
<223>  mature HtrA2(S306A, GVPS)

<400>  14

Met Gly Val Pro Ser Pro Pro Pro Ala Ser Pro Arg Ser Gln Tyr Asn
1               5                   10                  15

Phe Ile Ala Asp Val Val Glu Lys Thr Ala Pro Ala Val Val Tyr Ile

20                  25                  30

Glu Ile Leu Asp Arg His Pro Phe Leu Gly Arg Glu Val Pro Ile Ser
        35                  40                  45

Asn Gly Ser Gly Phe Val Val Ala Ala Asp Gly Leu Ile Val Thr Asn
    50                  55                  60

Ala His Val Val Ala Asp Arg Arg Arg Val Arg Val Arg Leu Leu Ser
65                  70                  75                  80

Gly Asp Thr Tyr Glu Ala Val Val Thr Ala Val Asp Pro Val Ala Asp
                85                  90                  95

Ile Ala Thr Leu Arg Ile Gln Thr Lys Glu Pro Leu Pro Thr Leu Pro
            100                 105                 110

Leu Gly Arg Ser Ala Asp Val Arg Gln Gly Glu Phe Val Val Ala Met
        115                 120                 125

Gly Ser Pro Phe Ala Leu Gln Asn Thr Ile Thr Ser Gly Ile Val Ser
    130                 135                 140

Ser Ala Gln Arg Pro Ala Arg Asp Leu Gly Leu Pro Gln Thr Asn Val
145                 150                 155                 160

Glu Tyr Ile Gln Thr Asp Ala Ala Ile Asp Phe Gly Asn Ala Gly Gly
                165                 170                 175

Pro Leu Val Asn Leu Asp Gly Glu Val Ile Gly Val Asn Thr Met Lys
            180                 185                 190

Val Thr Ala Gly Ile Ser Phe Ala Ile Pro Ser Asp Arg Leu Arg Glu
        195                 200                 205

Phe Leu His Arg Gly Glu Lys Lys Asn Ser Ser Ser Gly Ile Ser Gly
    210                 215                 220

Ser Gln Arg Arg Tyr Ile Gly Val Met Met Leu Thr Leu Ser Pro Ser
225                 230                 235                 240

Ile Leu Ala Glu Leu Gln Leu Arg Glu Pro Ser Phe Pro Asp Val Gln
                245                 250                 255

His Gly Val Leu Ile His Lys Val Ile Leu Gly Ser Pro Ala His Arg
            260                 265                 270

Ala Gly Leu Arg Pro Gly Asp Val Ile Leu Ala Ile Gly Glu Gln Met
        275                 280                 285

Val Gln Asn Ala Glu Asp Val Tyr Glu Ala Val Arg Thr Gln Ser Gln

290                 295                 300

Leu Ala Val Gln Ile Arg Arg Gly Arg Glu Thr Leu Thr Leu Tyr Val
305                 310                 315                 320

Thr Pro Glu Val Thr Glu
                325

<210>  15
<211>  2112
<212>  DNA
<213>  Homo sapiens

<220>
<221>  misc_feature
<223>  DNA that codes for CREBL1

<400>  15
atggcggagc tgatgctgct cagcgagatt gctgacccga cgcgtttctt caccgacaac      60

ctgcttagcc cggaggactg gggtctgcag aacagcacct tgtattctgg cctagatgaa     120

gtggccgagg agcagacgca gctcttccgt tgcccggagc aggatgtccc gtttgacggc     180

agctccctgg acgtggggat ggatgtcagc ccctctgagc ccccatggga actcctgccg     240

atcttcccag atcttcaggt gaagtctgag ccatcttccc cctgctcttc ctcctccctc     300

agctccgagt catcgcgtct ctccacagag ccatccagcg aggctcttgg ggtaggggag     360

gtgctccatg tgaagacaga gtccttggca cccccactgt gtctcctggg agatgaccca     420

acatcctcat ttgaaaccgt ccagatcaat gttatcccca cctctgatga ttcctcagat     480

gtccagacca agatagaacc tgtctctcca tgttcttccg tcaactctga ggcctccctg     540

ctctcagccg actcctccag ccaggctttt ataggagagg aggtcctgga agtgaagaca     600

gagtccctgt ccccttcagg atgcctcctg tgggatgtcc cagccccctc acttggagct     660

gtccagatca gcatgggccc atcccttgat ggctcctcag gcaaagccct gcccacccgg     720

aagccgccac tgcagcccaa acctgtagtg ctaaccactg tcccaatgcc atccagagct     780

gtgcctccca gcaccacagt ccttctgcag tccctcgtcc agccaccccc agtgtcccca     840

gttgtcctca tccagggtgc tattcgagtc cagcctgaag ggccggctcc ctctctacca     900

cggcctgaga ggaagagcat cgttcccgct cctatgcctg gaaactcctg cccgcctgaa     960

gtggatgcaa agctgctgaa gcggcagcag cgaatgatca agaaccggga gtcagcctgc    1020

cagtcccgga gaaagaagaa agagtatctg cagggactgg aggctcggct gcaagcagta    1080

ctggctgaca accagcagct ccgccgagag aatgctgccc tccggcggcg gctggaggcc    1140

ctgctggctg aaaacagcga gctcaagtta gggtctggaa acaggaaggt ggtctgcatc    1200

atggtcttcc ttctcttcat tgccttcaac tttggacctg tcagcatcag tgagcctcct    1260

tcagctccca tctctcctcg gatgaacaag ggggagcctc aaccccggag acacttgctg    1320

gggttctcag agcaagagcc agttcaggga gttgaacctc tccaggggtc ctcccagggc    1380

cctaaggagc cccagcccag ccccacagac cagcccagtt tcagcaacct gacagccttc 1440

cctgggggcg ccaaggagct actactaaga gacctagacc agctcttcct ctcctctgat 1500

tgccggcact tcaaccgcac tgagtccctg aggcttgctg acgagttgag tggctgggtc 1560

cagcgccacc agagaggccg gaggaagatc cctcagaggg cccaggagag acagaagtct 1620

cagccacgga agaagtcacc tccagttaag gcagtcccca tccaaccccc tggaccccca 1680

gaaagggatt ctgtgggcca gctgcaacta tatcgccacc cagaccgttc gcagccagca 1740

ttcttggatg caattgaccg acgggaagac acattttatg ttgtctcttt ccgaagggac 1800

cacctgctgc tcccagccat cagccacaac aagacctccc ggcccaagat gtccctggtg 1860

atgcctgcca tggcccccaa tgagaccctg tcaggccgtg ggccccgggg ggactatgag 1920

gagatgatgc agatcgagtg tgaggtcatg gacaccaggg tgattcacat caagacctcc 1980

acagtgcccc cctcgctccg aaaacagcca tccccaaccc caggcaatgc cacaggtggc 2040

cccttgccag tctctgcagc cagccaggcc caccaggcct cccaccagcc cctctacctc 2100

aatcatccct aa 2112

<210> 16
<211> 703
<212> PRT
<213> Homo sapiens

<220>
<221> misc_feature
<223> CREBL1

<400> 16

Met Ala Glu Leu Met Leu Leu Ser Glu Ile Ala Asp Pro Thr Arg Phe
1               5                   10                  15

Phe Thr Asp Asn Leu Leu Ser Pro Glu Asp Trp Gly Leu Gln Asn Ser
            20                  25                  30

Thr Leu Tyr Ser Gly Leu Asp Glu Val Ala Glu Glu Gln Thr Gln Leu
        35                  40                  45

Phe Arg Cys Pro Glu Gln Asp Val Pro Phe Asp Gly Ser Ser Leu Asp
    50                  55                  60

Val Gly Met Asp Val Ser Pro Ser Glu Pro Pro Trp Glu Leu Leu Pro
65                  70                  75                  80

Ile Phe Pro Asp Leu Gln Val Lys Ser Glu Pro Ser Ser Pro Cys Ser
                85                  90                  95

Ser Ser Ser Leu Ser Ser Glu Ser Ser Arg Leu Ser Thr Glu Pro Ser
            100                 105                 110

Ser Glu Ala Leu Gly Val Gly Glu Val Leu His Val Lys Thr Glu Ser

115                 120                 125

Leu Ala Pro Pro Leu Cys Leu Leu Gly Asp Asp Pro Thr Ser Ser Phe
    130                 135                 140

Glu Thr Val Gln Ile Asn Val Ile Pro Thr Ser Asp Asp Ser Ser Asp
145                 150                 155                 160

Val Gln Thr Lys Ile Glu Pro Val Ser Pro Cys Ser Ser Val Asn Ser
                165                 170                 175

Glu Ala Ser Leu Leu Ser Ala Asp Ser Ser Ser Gln Ala Phe Ile Gly
            180                 185                 190

Glu Glu Val Leu Glu Val Lys Thr Glu Ser Leu Ser Pro Ser Gly Cys
        195                 200                 205

Leu Leu Trp Asp Val Pro Ala Pro Ser Leu Gly Ala Val Gln Ile Ser
    210                 215                 220

Met Gly Pro Ser Leu Asp Gly Ser Ser Gly Lys Ala Leu Pro Thr Arg
225                 230                 235                 240

Lys Pro Pro Leu Gln Pro Lys Pro Val Val Leu Thr Thr Val Pro Met
                245                 250                 255

Pro Ser Arg Ala Val Pro Pro Ser Thr Thr Val Leu Leu Gln Ser Leu
            260                 265                 270

Val Gln Pro Pro Pro Val Ser Pro Val Val Leu Ile Gln Gly Ala Ile
        275                 280                 285

Arg Val Gln Pro Glu Gly Pro Ala Pro Ser Leu Pro Arg Pro Glu Arg
    290                 295                 300

Lys Ser Ile Val Pro Ala Pro Met Pro Gly Asn Ser Cys Pro Pro Glu
305                 310                 315                 320

Val Asp Ala Lys Leu Leu Lys Arg Gln Gln Arg Met Ile Lys Asn Arg
                325                 330                 335

Glu Ser Ala Cys Gln Ser Arg Arg Lys Lys Lys Glu Tyr Leu Gln Gly
            340                 345                 350

Leu Glu Ala Arg Leu Gln Ala Val Leu Ala Asp Asn Gln Gln Leu Arg
        355                 360                 365

Arg Glu Asn Ala Ala Leu Arg Arg Arg Leu Glu Ala Leu Leu Ala Glu
    370                 375                 380

Asn Ser Glu Leu Lys Leu Gly Ser Gly Asn Arg Lys Val Val Cys Ile

385                 390                 395                 400

Met Val Phe Leu Leu Phe Ile Ala Phe Asn Phe Gly Pro Val Ser Ile
                405                 410                 415

Ser Glu Pro Pro Ser Ala Pro Ile Ser Pro Arg Met Asn Lys Gly Glu
            420                 425                 430

Pro Gln Pro Arg Arg His Leu Leu Gly Phe Ser Glu Gln Glu Pro Val
        435                 440                 445

Gln Gly Val Glu Pro Leu Gln Gly Ser Ser Gln Gly Pro Lys Glu Pro
    450                 455                 460

Gln Pro Ser Pro Thr Asp Gln Pro Ser Phe Ser Asn Leu Thr Ala Phe
465                 470                 475                 480

Pro Gly Gly Ala Lys Glu Leu Leu Leu Arg Asp Leu Asp Gln Leu Phe
                485                 490                 495

Leu Ser Ser Asp Cys Arg His Phe Asn Arg Thr Glu Ser Leu Arg Leu
            500                 505                 510

Ala Asp Glu Leu Ser Gly Trp Val Gln Arg His Gln Arg Gly Arg Arg
        515                 520                 525

Lys Ile Pro Gln Arg Ala Gln Glu Arg Gln Lys Ser Gln Pro Arg Lys
    530                 535                 540

Lys Ser Pro Pro Val Lys Ala Val Pro Ile Gln Pro Pro Gly Pro Pro
545                 550                 555                 560

Glu Arg Asp Ser Val Gly Gln Leu Gln Leu Tyr Arg His Pro Asp Arg
                565                 570                 575

Ser Gln Pro Ala Phe Leu Asp Ala Ile Asp Arg Arg Glu Asp Thr Phe
            580                 585                 590

Tyr Val Val Ser Phe Arg Arg Asp His Leu Leu Leu Pro Ala Ile Ser
        595                 600                 605

His Asn Lys Thr Ser Arg Pro Lys Met Ser Leu Val Met Pro Ala Met
    610                 615                 620

Ala Pro Asn Glu Thr Leu Ser Gly Arg Gly Ala Pro Gly Asp Tyr Glu
625                 630                 635                 640

Glu Met Met Gln Ile Glu Cys Glu Val Met Asp Thr Arg Val Ile His
                645                 650                 655

Ile Lys Thr Ser Thr Val Pro Pro Ser Leu Arg Lys Gln Pro Ser Pro

660                 665                 670

Thr Pro Gly Asn Ala Thr Gly Gly Pro Leu Pro Val Ser Ala Ala Ser
        675                 680                 685

Gln Ala His Gln Ala Ser His Gln Pro Leu Tyr Leu Asn His Pro
    690                 695                 700

<210>  17
<211>  2013
<212>  DNA
<213>  Homo sapiens

<220>
<221>  misc_feature
<223>  DNA that codes for ATF6

<400>  17
atgggggagc cggctggggt tgccggcacc atggagtcac cttttagccc gggactcttt      60

cacaggctgg atgaagattg ggattctgct ctctttgctg aactcggtta tttcacagac     120

actgatgagc tgcaattgga agcagcaaat gagacgtatg aaaacaattt tgataatctt     180

gattttgatt tggatttgat gccttgggag tcagacattt gggacatcaa caaccaaatc     240

tgtacagtta aagatattaa ggcagaacct cagccacttt ctccagcctc ctcaagttat     300

tcagtctcgt ctcctcggtc agtggactct tattcttcaa ctcagcatgt tcctgaggag     360

ttggatttgt cttctagttc tcagatgtct cccctttcct tatatggtga aaactctaat     420

agtctctctt cagcggagcc actgaaggaa gataagcctg tcactggtcc taggaacaag     480

actgaaaatg gactgactcc aaagaaaaaa attcaggtga attcaaaacc ttcaattcag     540

cccaagcctt tattgcttcc agcagcaccc aagactcaaa caaactccag tgttccagca     600

aaaaccatca ttattcagac agtaccaacg cttatgccat tggcaaagca gcaaccaatt     660

atcagtttac aacctgcacc cactaaaggc cagacggttt tgctgtctca gcctactgtg     720

gtacaacttc aagcacctgg agttctgccc tctgctcagc cagtccttgc tgttgctggg     780

ggagtcacac agctccctaa tcacgtggtg aatgtggtac cagccccttc agcgaatagc     840

ccagtgaatg gaaaactttc cgtgactaaa cctgtcctac aaagtaccat gagaaatgtc     900

ggttcagata ttgctgtgct aaggagacag caacgtatga taaaaaatcg agaatccgct     960

tgtcagtctc gcaagaagaa gaaagaatat atgctagggt tagaggcgag attaaaggct    1020

gccctctcag aaaacgagca actgaagaaa gaaaatggaa cactgaagcg gcagctggat    1080

gaagttgtgt cagagaacca gaggcttaaa gtccctagtc caaagcgaag agttgtctgt    1140

gtgatgatag tattggcatt tataatactg aactatggac ctatgagcat gttggaacag    1200

gattccagga gaatgaaccc tagtgtgagc cctgcaaatc aaaggaggca ccttctagga    1260

ttttctgcta aagaggcaca ggacacatca gatggtatta tccagaaaaa cagctacaga    1320

tatgatcatt ctgtttcaaa tgacaaagcc ctgatggtgc taactgaaga accattgctt    1380

tacattcctc cacctccttg tcagccccta attaacacaa cagagtctct caggttaaat 1440

catgaacttc gaggatgggt tcatagacat gaagtagaaa ggaccaagtc aagaagaatg 1500

acaaataatc aacagaaaac ccgtattctt cagggtgctc tggaacaggg ctcaaattct 1560

cagctgatgg ctgttcaata cacagaaacc actagtagta tcagcaggaa ctcagggagt 1620

gagctacaag tgtattatgc ttcacccaga agttatcaag acttttttga agccatccgc 1680

agaaggggag acacatttta tgttgtgtca tttcgaaggg atcacctgct gttaccagct 1740

accacccata acaagaccac aagaccaaaa atgtcaattg tgttaccagc aataaacata 1800

aatgagaatg tgatcaatgg gcaggactac gaagtgatga tgcagattga ctgtcaggtg 1860

atggacacca ggatcctcca tatcaaaagt tcgtcagttc ctccttacct ccgagatcag 1920

cagaggaatc aaaccaacac cttctttggc tcccctcccg cagccacaga ggcaacccac 1980

gttgtcagca ccatccctga gtcattacaa tag 2013

<210> 18
<211> 670
<212> PRT
<213> Homo sapiens

<220>
<221> misc_feature
<223> ATF6

<400> 18

<210>  19

<211>  36
<212>  DNA
<213>  Artificial

<220>
<223>  Designed polynucleotide based on the base sequence of SEQ ID NO:3
       for use as a primer to obtain mature HtrA2 DNA

<400>  19
catatggccg tccctagccc gccgcccgct tctccc                                36


<210>  20
<211>  35
<212>  DNA
<213>  Artificial

<220>
<223>  Designed polynucleotide based on the base sequence of SEQ ID NO:3
       for use as a primer to obtain mature HtrA2 DNA

<400>  20
ctcgagttct gtgacctcag gggtcacata taagg                                  35


<210>  21
<211>  40
<212>  DNA
<213>  Artificial

<220>
<223>  Designed polynucleotide based on the base sequence of SEQ ID NO:3
       for use as a primer to obtain mature HtrA2(S306A) DNA

<400>  21
gctattgatt ttggaaacgc tggaggtccc ctggttaacc                            40


<210>  22
<211>  40
<212>  DNA
<213>  Artificial

<220>
<223>  Designed polynucleotide based on the base sequence of SEQ ID NO:3
       for use as a primer to obtain mature HtrA2(S306A) DNA

<400>  22
ggttaaccag gggacctcca gcgtttccaa aatcaatagc                            40


<210>  23
<211>  27
<212>  DNA
<213>  Artificial

<220>
<223>  Designed polynucleotide based on the base sequence of SEQ ID
       NO:15 for use as a primer to obtain CREBL1 DNA

<400>  23
gcgaattcgc catggcggag ctgatgc                                          27


<210>  24
<211>  28
<212>  DNA
<213>  Artificial

<220>
<223>  Designed polynucleotide based on the base sequence of SEQ ID
       NO:15 for use as a primer to obtain CREBL1 DNA

<400>  24
gcctcgaggg gatgattgag gtagaggg                                          28


<210>  25
<211>  30
<212>  DNA
<213>  Artificial

<220>
<223>  Designed polynucleotide based on the base sequence of SEQ ID
       NO:15 for use as a primer to obtain CREBL1 DNA

<400>  25
gcggatcccg cggagctgat gctgctcagc                                        30


<210>  26
<211>  33
<212>  DNA
<213>  Artificial

<220>
<223>  Designed polynucleotide based on the base sequence of SEQ ID
       NO:15 for use as a primer to obtain CREBL1 DNA

<400>  26
cctcgaggtt tagggatgat tgaggtagag ggg                                    33


<210>  27
<211>  30
<212>  DNA
<213>  Artificial

<220>
<223>  Designed polynucleotide based on the base sequence of SEQ ID
       NO:17 for use as a primer to obtain ATF6 DNA

<400>  27
agttccaggg aaaaggaact tgtgaaatgg                                        30


<210>  28
<211>  30
<212>  DNA
<213>  Artificial

<220>
<223>  Designed polynucleotide based on the base sequence of SEQ ID
       NO:17 for use as a primer to obtain ATF6 DNA

<400>  28
acgctcagtt ttccacatag ctgcgggtgc                                        30


<210>  29
<211>  39
<212>  DNA
<213>  Artificial

<220>
<223>  Designed polynucleotide based on the base sequence of SEQ ID

NO:17 for use as a primer to obtain ATF6 DNA

<400> 29
aaagatatca tgggggagcc ggctggggtt gccggcacc 39

<210> 30
<211> 39
<212> DNA
<213> Artificial

<220>
<223> Designed polynucleotide based on the base sequence of SEQ ID
NO:17 for use as a primer to obtain ATF6 DNA

<400> 30
aaactcgagc tattgtaatg actcagggat ggtgctgac 39

<210> 31
<211> 39
<212> DNA
<213> Artificial

<220>
<223> Designed polynucleotide based on the base sequence of SEQ ID
NO:17 for use as a primer to obtain ATF6 DNA

<400> 31
aaaagatcta tgggggagcc ggctggggtt gccggcacc 39

<210> 32
<211> 36
<212> DNA
<213> Artificial

<220>
<223> Designed polynucleotide based on the base sequence of SEQ ID NO:3
for use as a primer to obtain mature HtrA2(delta AVPS) DNA

<400> 32
gagctcatgc cgccgcccgc ttctccccgg agtcag 36

<210> 33
<211> 36
<212> DNA
<213> Artificial

<220>
<223> Designed polynucleotide based on the base sequence of SEQ ID NO:3
for use as a primer to obtain mature HtrA2(GVPS) DNA

<400> 33
gagctcatgg gcgtccctag cccgccgccc gcttct 36

<210> 34
<211> 1398
<212> DNA
<213> Homo sapiens

<220>
<221> misc_feature
<223> DNA that codes for HNF-4alpha

<400>  34
atggacatgg ccgactacag tgctgcactg gacccagcct acaccaccct ggaatttgag      60

aatgtgcagg tgttgacgat gggcaatgac acgtccccat cagaaggcac caacctcaac     120

gcgcccaaca gcctgggtgt cagcgccctg tgtgccatct gcggggaccg ggccacgggc     180

aaacactacg gtgcctcgag ctgtgacggc tgcaagggct tcttccggag gagcgtgcgg     240

aagaaccaca tgtactcctg cagatttagc cggcagtgcg tggtggacaa agacaagagg     300

aaccagtgcc gctactgcag gctcaagaaa tgcttccggg ctggcatgaa gaaggaagcc     360

gtccagaatg agcgggaccg gatcagcact cgaaggtcaa gctatgagga cagcagcctg     420

ccctccatca atgcgctcct gcaggcggag gtcctgtccc gacagatcac ctcccccgtc     480

tccgggatca acggcgacat tcgggcgaag aagattgcca gcatcgcaga tgtgtgtgag     540

tccatgaagg agcagctgct ggttctcgtt gagtgggcca agtacatccc agctttctgc     600

gagctccccc tggacgacca ggtggccctg ctcagagccc atgctggcga gcacctgctg     660

ctcggagcca ccaagagatc catggtgttc aaggacgtgc tgctcctagg caatgactac     720

attgtccctc ggcactgccc ggagctggcg gagatgagcc gggtgtccat acgcatcctt     780

gacgagctgg tgctgccctt ccaggagctg cagatcgatg acaatgagta tgcctacctc     840

aaagccatca tcttctttga cccagatgcc aaggggctga gcgatccagg gaagatcaag     900

cggctgcgtt cccaggtgca ggtgagcttg gaggactaca tcaacgaccg ccagtatgac     960

tcgcgtggcc gctttggaga gctgctgctg ctgctgccca ccttgcagag catcacctgg    1020

cagatgatcg agcagatcca gttcatcaag ctcttcggca tggccaagat tgacaacctg    1080

ttgcaggaga tgctgctggg agggtccccc agcgatgcac cccatgccca ccaccccctg    1140

caccctcacc tgatgcagga acatatggga accaacgtca tcgttgccaa cacaatgccc    1200

actcacctca gcaacggaca gatgtgtgag tggccccgac ccaggggaca ggcagccacc    1260

cctgagaccc cacagccctc accgccaggt ggctcagggt ctgagcccta taagctcctg    1320

ccgggagccg tcgccacaat cgtcaagccc ctctctgcca tcccccagcc gaccatcacc    1380

aagcaggaag ttatctag                                                  1398

<210>  35
<211>  465
<212>  PRT
<213>  Homo sapiens

<220>
<221>  misc_feature
<223>  HNF-4alpha

<400>  35

Met Asp Met Ala Asp Tyr Ser Ala Ala Leu Asp Pro Ala Tyr Thr Thr
1               5                   10                  15

Leu Glu Phe Glu Asn Val Gln Val Leu Thr Met Gly Asn Asp Thr Ser
            20                  25                  30

Pro Ser Glu Gly Thr Asn Leu Asn Ala Pro Asn Ser Leu Gly Val Ser
        35                  40                  45

Ala Leu Cys Ala Ile Cys Gly Asp Arg Ala Thr Gly Lys His Tyr Gly
    50                  55                  60

Ala Ser Ser Cys Asp Gly Cys Lys Gly Phe Phe Arg Arg Ser Val Arg
65                  70                  75                  80

Lys Asn His Met Tyr Ser Cys Arg Phe Ser Arg Gln Cys Val Val Asp
                85                  90                  95

Lys Asp Lys Arg Asn Gln Cys Arg Tyr Cys Arg Leu Lys Lys Cys Phe
            100                 105                 110

Arg Ala Gly Met Lys Lys Glu Ala Val Gln Asn Glu Arg Asp Arg Ile
        115                 120                 125

Ser Thr Arg Arg Ser Ser Tyr Glu Asp Ser Ser Leu Pro Ser Ile Asn
    130                 135                 140

Ala Leu Leu Gln Ala Glu Val Leu Ser Arg Gln Ile Thr Ser Pro Val
145                 150                 155                 160

Ser Gly Ile Asn Gly Asp Ile Arg Ala Lys Lys Ile Ala Ser Ile Ala
                165                 170                 175

Asp Val Cys Glu Ser Met Lys Glu Gln Leu Leu Val Leu Val Glu Trp
            180                 185                 190

Ala Lys Tyr Ile Pro Ala Phe Cys Glu Leu Pro Leu Asp Asp Gln Val
        195                 200                 205

Ala Leu Leu Arg Ala His Ala Gly Glu His Leu Leu Leu Gly Ala Thr
    210                 215                 220

Lys Arg Ser Met Val Phe Lys Asp Val Leu Leu Leu Gly Asn Asp Tyr
225                 230                 235                 240

Ile Val Pro Arg His Cys Pro Glu Leu Ala Glu Met Ser Arg Val Ser
                245                 250                 255

Ile Arg Ile Leu Asp Glu Leu Val Leu Pro Phe Gln Glu Leu Gln Ile
            260                 265                 270

Asp Asp Asn Glu Tyr Ala Tyr Leu Lys Ala Ile Ile Phe Phe Asp Pro
        275                 280                 285

Asp Ala Lys Gly Leu Ser Asp Pro Gly Lys Ile Lys Arg Leu Arg Ser
    290                 295                 300

Gln Val Gln Val Ser Leu Glu Asp Tyr Ile Asn Asp Arg Gln Tyr Asp
305                 310                 315                 320

Ser Arg Gly Arg Phe Gly Glu Leu Leu Leu Leu Leu Pro Thr Leu Gln
                325                 330                 335

Ser Ile Thr Trp Gln Met Ile Glu Gln Ile Gln Phe Ile Lys Leu Phe
            340                 345                 350

Gly Met Ala Lys Ile Asp Asn Leu Leu Gln Glu Met Leu Leu Gly Gly
        355                 360                 365

Ser Pro Ser Asp Ala Pro His Ala His His Pro Leu His Pro His Leu
    370                 375                 380

Met Gln Glu His Met Gly Thr Asn Val Ile Val Ala Asn Thr Met Pro
385                 390                 395                 400

Thr His Leu Ser Asn Gly Gln Met Cys Glu Trp Pro Arg Pro Arg Gly
                405                 410                 415

Gln Ala Ala Thr Pro Glu Thr Pro Gln Pro Ser Pro Pro Gly Gly Ser
            420                 425                 430

Gly Ser Glu Pro Tyr Lys Leu Leu Pro Gly Ala Val Ala Thr Ile Val
        435                 440                 445

Lys Pro Leu Ser Ala Ile Pro Gln Pro Thr Ile Thr Lys Gln Glu Val
    450                 455                 460

Ile
465

## Description

### TECHNICAL FIELD

The present invention relates to a method for inhibiting the degradation of at least one of CREBL1 (cAMP responsive element binding protein-like 1), ATF6 (activating transcription factor 6), and HNF-4α (hepatocyte nuclear factor 4α), and a method for preventing and/or treating diabetes, comprising inhibiting the degradation by HtrA2 (high temperature requirement protein A2) of at least one of CREBL1, ATF6, and HNF-4α Particularly, the present invention relates to a method for inhibiting the degradation of at least one of CREBL1, ATF6, and HNF-4α, comprising inhibiting the function of HtrA2 (for example, inhibiting the cleavage by HtrA2 of at least one of CREBL1, ATF6, and HNF-4α or inhibiting the interaction of at least one of CREBL1, ATF6, and HNF-4α with HtrA2). Further, the present invention relates to a method for preventing and/or treating diabetes comprising using the aforementioned degradation method. Further, the present invention relates to a method for preventing and/or treating diabetes comprising using one or more compounds that inhibit the degradation by HtrA2 of at least one of CREBL1, ATF6, and HNF-4α, and an agent for preventing and/or treating diabetes comprising one or more compounds. Further, the present invention relates to a method of identifying a compound that inhibits the degradation of at least one of CREBL1, ATF6, and HNF-4α Further, the present invention relates to a method of identifying a compound that is to be an active ingredient for an agent for preventing and/or treating diabetes. Further, the present invention relates to a method for inhibiting cell death comprising inhibiting the degradation by HtrA2 of CREBL1 and/or ATF6. Further, the present invention relates to a method for inhibiting cell death comprising using one or more compounds that inhibit the degradation by HtrA2 of CREBL1 and/or ATF6, and to an agent for inhibiting cell death comprising one or more compounds. Further, the present invention relates to a method for preventing and/or treating type 2 diabetes comprising inhibiting the degradation by HtrA2 of HNF-4α Further, the present invention relates to an agent for preventing and/or treating type 2 diabetes comprising one or more compounds that inhibit the degradation by HtrA2 of HNF-4α Further, the present invention relates to a reagent kit comprising at least one selected from the group consisting of HtrA2, a polynucleotide encoding HtrA2, and a vector containing the polynucleotide encoding HtrA2; and at least one selected from the group consisting of CREBL1, ATF6, BNF-4α, a polynucleotide encoding CREBL1, ATF6, or HNF-4α and a vector containing the polynucleotide encoding CREBL1, ATF6, or HNF-4α

### BACKGROUND OF INVENTION

Organisms are taking self defense against stresses formed on the environmentals, by regulating the survival and apoptosis (cell death) of cells, while the disturbed regulation of the survival and apoptosis-controlling mechanism may cause excessive cell death to induce various disorders (Non-Patent Reference 1).

In recent years, researches have been remarkable progressed on mitochondria and endoplasmic reticulum as sites for perceiving and regulating stresses. It has been reported that HtrA2 protein (hereinafter referred to as HtrA2) located at a mitochondrial membrane translocates from the mitochondrial membrane to cytoplasm due to stress such as UV and heat shock (Non-Patent References 2-5), thereby induces two types of cell death, that is, caspase dependent cell death and caspase independent cell death (Non-Patent Reference 6). HtrA2 precursor protein (SEQ ID NO: 2) converts to the mature type (SEQ ID NO: 4) by being cleaved the N-terminal 133 amino acids thereof and then translocates from mitochondria to cytoplasm. The mature HtrA2 has a protease activity. Herein after, HtrA2 with the protease activity may be referred to as active HtrA2.

It is known that caspase independent cell death is depending on the feature of HtrA2 itself as a serine protease and that caspase independent cell death is not triggered by the HtrA2 mutant with 306^{th} serine in the amino acid sequence of the precursor protein thereof (this position is corresponding to 174^{th} in mature type) substituted with alanine (Non-Patent References 3-5). Such HtrA2 mutant has no protease activity. Herein after, HtrA2 without the protease activity may be referred to as inactive HtrA2. As mentioned, HtrA2 is a factor that plays important roles in the mechanism of inducing caspase independent cell death.

The References cited in the specification are listed as follows:
Non-patent Reference 1: "Saibou Kougaku (Cell Technology) ", 2002, Vol.21, No.4, p.360-394.
Non-patent Reference 2: Yamaguchi H. et al., "Cancer Research", 2003, Vol.63, p.1483-1489.
Non-patent Reference 3: Suzuki Y. et al., "Molecular Cell", 2001, Vol.8, p.613-621.
Non-patent Reference 4: Hegde R.et al., "The Journal of Biological Chemistry", 2002, Vol.277, p.432-438.
Non-patent Reference 5: Martins L.M. et al., "The Journal of Biological Chemistry", 2002, Vol.277, p.439-444.
Non-patent Reference 6: "Jikken Igaku (Experimental Medicine) ", 2002, Vol.20, No.1, p.73-75.
Non-patent Reference 7: "Naika", 2003, Vol.91, No. 1, p.63-67.
Non-patent Reference8: Kaufman R.J. et al., "The Journal of Clinical Investigation", 2002, Vol.110, p.1389-1398.
Non-patent Reference 9: Haze K. et al., "The Biochemical Journal", 2001, Vol.355, p.19-28.
Non-patent Reference 10: "Journal of Molecular Endocrinology", 2001, Vol.27, No.1, p.11-29.
Non-patent Reference 11: "Rinsho Byori (The Japanese Journal of Clinical Pathology)", 2001, Vol.49, No.2, p.161-164.
Non-patent Reference 12: Ulmer K.M., "Science", 1983, Vol.219, p.666-671.
Non-patent Reference 13: "PEPUTIDO GOUSEI", Maruzen Co., Ltd., 1975.
Non-patent Reference 14: "Peptide Synthesis", Interscience, New York, 1996.

### DISCLOSURE OF THE INVENTION

### [PROBLEM TO BE SOLVED BY THE INVENTION]

In recent years, it has been reported that endoplasmic reticulum stress causes pancreatic *β* cell death, resulting in the onset of diabetes. The pancreatic *β* cell is in charge of the production and secretion of insulin, which has highly developed endoplasmic reticulum and is highly sensitive to endoplasmic reticulum stress. In other words, it is considered that collapse of the defense system in response to endoplasmic reticulum stress causes *β* cell loss by cell death, resulting in insulin secretion failure that results in aggravation of diabetes (Non-Patent Reference 7). In the meantime, it is also reported that endoplasmic reticulum stress enhances the expression ofHtrA2. From these facts, it is believed that HtrA2 is likely to be a new target of diabetes and that inhibition of cell death due to endoplasmic reticulum stress may become a novel target for drug discovery.

Under the circumstances where a mechanism of caspase independent cell death caused by HtrA2 and a substrate of HtrA2 have not yet been clarified, an object of the present invention is to find out a protein that interacts with HtrA2 and to provide a means that allows prevention and/or treatment of disorders attributable to the degradation of the protein by HtrA2.

### [MEANS FOR SOLVING THE PROBLEM]

The present inventors have concentrated intensively efforts to solve the aforementioned problem. We predicted *in-silico* that HtrA2 interacts with CREBL1 and HNF-4α, and then found experimentally that CREBL1, ATF6 that is a family member of CREBL1, and HNF-4α, were degraded by active HtrA2. Thus, we achieved the present invention.

Namely, the present invention relates to a method for inhibiting the degradation of at least one of CREBL1 (cAMP responsive element binding protein-like 1), ATF6 (activating transcription factor 6), and HNF-4α (hepatocyte nuclear factor-4α), comprising inhibiting the function of HtrA2 (high temperature requirement protein A2).

The present invention also relates to the aforementioned method for inhibiting the degradation of at least one of CREBL1, ATF6, and HNF-4α, in which to inhibit the function of HtrA2 is to inhibit the cleavage by HtrA2 of at least one of CREBL1, ATF6, and HNF-4α, wherein to inhibit the cleavage by HtrA2 of CREBL1 brings inhibition of the degradation of CREBL1, to inhibit the cleavage by HtrA2 of ATF6 brings inhibition of the degradation of ATF6, and to inhibit the cleavage by HtrA2 of HNF-4α brings inhibition of the degradation of HNF-4α.

The present invention further relates to the aforementioned method for inhibiting the degradation of at least one of CREBL1, ATF6, and HNF-4α, in which to inhibit the function of HtrA2 is to inhibit the interaction of the HtrA2 with at least one of CREBL1, ATF6, and HNF-4α, wherein to inhibit the interaction of the HtrA2 with CREBL1 brings inhibition of the degradation of CREBL1, to inhibit the interaction of the HtrA2 with ATF6 brings inhibition of the degradation of ATF6, and to inhibit the interaction of the HtrA2 with HNF-4α brings inhibition of the degradation ofHNF-4o.

The present invention still further relates to a method for preventing and/or treating diabetes, comprising inhibiting the degradation by HtrA2 of at least one of CREBL1, ATF6, and HNF-4α

The present invention also relates to a method for preventing and/or treating diabetes, comprising using the aforementioned method for inhibiting the degradation of at least one of CREBL1, ATF6, and HNF-4α

The present invention further relates to a method for preventing and/or treating diabetes, comprising using one or more compounds that inhibit the degradation by HtrA2 of at least one of CREBL1, ATF6, and HNF-4α.

The present invention still further relates to an agent for preventing and/or treating diabetes, comprising one or more compounds that inhibit the degradation by HtrA2 of at least one of CREBL1, ATF6, and HNF-4α

The present invention also relates to a method of identifying a compound that inhibits the degradation by HtrA2 of at least one of CREBL1, ATF6, and HNF-4α comprising contacting at least one of CREBL1, ATF6, and HNF-4α and/or HtrA2 with a compound (a test compound) under conditions that allow the degradation by HtrA2 of at least one of CREBL1, ATF6, and HNF-4α, introducing a system using a signal and/or a marker capable of detecting at least one of CREBL1, ATF6, and HNF-4α, detecting the presence or absence and/or change of the signal and/or the marker; and determining whether the test compound inhibits the degradation of at least one of CREBL1, ATF6, and HNF-4α.

The present invention further relates to a method of identifying a compound that inhibits the degradation by HtrA2 of at least one of CREBL1, ATF6, and HNF-4α, comprising contacting at least one of CREBL1, ATF6, and HNF-4α and/or HtrA2 with a compound (a test compound) under conditions that allow the degradation by HtrA2 of at least one of CREBL1, ATF6, and HNF-4α, detecting the presence or absence of at least one of CREBL1, ATF6, and HNF-4α, and/or measuring the change of the amount thereof; or detecting the presence or absence of the degradation product of at least one of CREBL1, ATF6, and HNF-4α, and/or measuring the change of the amount thereof ; and determining whether the test compound inhibits the degradation of at least one of CREBL1, ATF6, and HNF-4α.

The present invention still further relates to the aforementioned method, wherein the method of identifying a compound that inhibits the degradation by HtrA2 of at least one of CREBL1, ATF6, and HNF-4α is a method of identifying a compound that is an active ingredient in an agent for preventing and/or treating diabetes.

The present invention also relates to a method for inhibiting cell death, comprising inhibiting the degradation by HtrA2 of CREBL1 and/or ATF6.

The present invention further relates to a method for inhibiting cell death, comprising using one or more compounds that inhibit the degradation by HtrA2 of CREBL1 and/or ATF6.

The present invention still further relates to the aforementioned method for inhibiting cell death, in which the cell death is cell death of a pancreatic *β* cell.

The present invention also relates to an agent for inhibiting cell death, comprising one or more compounds that inhibit the degradation by HtrA2 of CREBL1 and/or ATF6.

The present invention further relates to the aforementioned agent for inhibiting cell death, wherein the cell death is cell death of a pancreatic *β* cell.

The present invention still further relates to a method for preventing and/or treating diabetes, comprising using the aforementioned method for inhibiting cell death.

The present invention also relates to a method for preventing and/or treating type 2 diabetes, comprising inhibiting the degradation by HtrA2 of HNF-4α.

The present invention further relates to an agent for preventing and/or treating type 2 diabetes, comprising one or more compounds that inhibit the degradation by HtrA2 of HNF-4α.

The present invention still further relates to a reagent kit, comprising at least one selected from the group consisting of HtrA2, a polynucleotide encoding HtrA2, and a vector containing the polynucleotide encoding HtrA2; and at least one selected from the group consisting of CREBL1, ATF6, HNF-4α, a polynucleotide encoding CREBL1, ATF6, or HNF-4α, and a vector containing the polynucleotide encoding CREBL1, ATF6, or HNF-4α.

### [ADVANTAGE OF THE INVENTION]

According to the present invention, to inhibit the degradation by HtrA2 of at least one of CREBL1, ATF6, and HNF-4α enables to inhibit the reduction or disappearance of the amount of at least one of these proteins, and as result, enables to prevent and/or treat diabetes.

It is considered that both CREBL1 and ATF6 perceive the endoplasmic reticulum stress to induce transcription of chaperon genes and involve in the recovery from the endoplasmic reticulum stress in cells and survival of the cells (Non-Patent References 8 and 9). It has also been reported that the endoplasmic reticulum stress causes pancreatic j8 cell death, and thereby promotes diabetes (Non-Patent Reference 7). From these facts, it is believed that the degradation of CREBL1 and/or ATF6 by HtrA2 causes the reduction or disappearance of the amount of these proteins, and thereby induces the enhanced cell death due to stress and the like, such as pancreatic *β* cell death, resulting in the promoted diabetes. Therefore, to inhibit the degradation by HtrA2 of CREBL1 and/or ATF6 and thereby to inhibit the reduction or disappearance of the amount of these proteins enable to inhibit cell death (such as pancreatic *β* cell death), and as result, enables to prevent and/or treat diabetes.

It has been clarified that loss of HNF-4α function causes disordered glucose metabolism and that HNF-4α gene is a causative gene for genetic type 2 diabetes MODY1 (Non-Patent References 10 and 11). From these facts, it is believed that the reduction or disappearance of the amount of HNF-4α causes disordered glucose metabolism, which results in aggravation of diabetes (for example, type 2 diabetes). In other words, it is believed that the degradation of HNF-4α by HtrA2 causes the reduction or disappearance of the amount of HNF-4α, thereby to give disordered glucose metabolism, which results in aggravation of diabetes (for example, type 2 diabetes). Therefore, to inhibit the degradation by HtrA2 of HNF-4α and thereby to inhibit the reduction or disappearance of the amount of HNF-4α enable to normalize the glucose metabolism, and as result, enable to prevent and/or treat diabetes (for example, type 2 diabetes).

It is believed that inhibition of the degradation by HtrA2 of CREBL1 and/or ATF6 and inhibition of the degradation by HtrA2 of HNF-4α act in preventing and/or treating diabetes through their respective different mechanisms. Therefore, to inhibit both the degradation by HtrA2 of CREBL1 and/or ATF6 and the degradation by HtrA2 of HNF-4α enables to achieve the prevention and/or treatment of diabetes through both of their respective different mechanisms, thereby to provide a higher effect.

According to the present invention, a method of identifying a compound that inhibits the degradation by HtrA2 of at least one of CREBL1, ATF6 and HNF-4α can be also carried out. The compound obtained by the identification method can be used for preventing and/or treating diabetes.

### BRIEF DESCRIPTION OF THE DRAWINGS

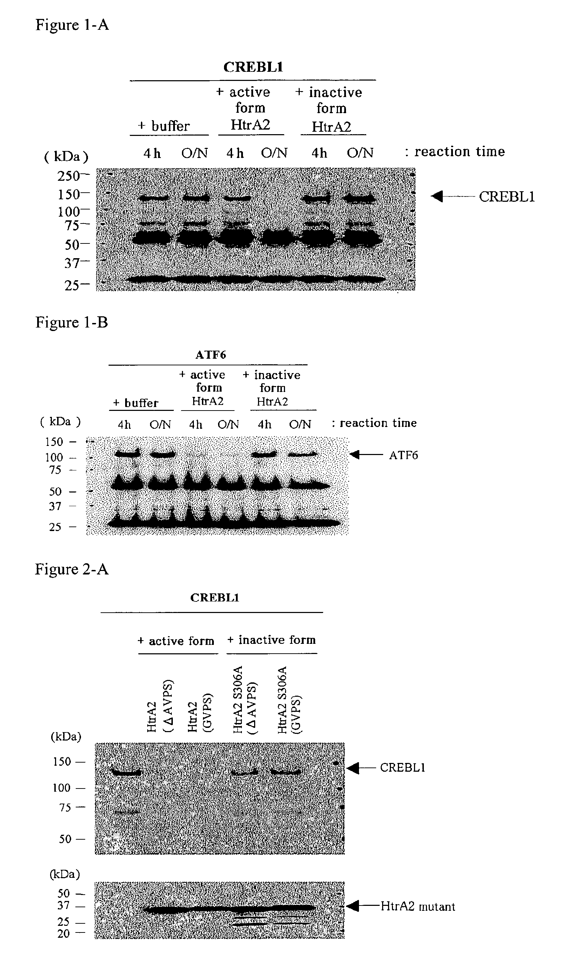

Fig. 1-A shows that CREBL1 was degraded in vitro by active HtrA2 (mature HtrA2). The band of CREBL1 was reduced significantly with the reaction of active HtrA2 with CREBL1 for overnight (O/N). On the other hand, the degradation of CREBL1 by inactive HtrA2 (mature HtrA2 (S306A)) was not observed (Example 2).

Fig. 1-B shows that ATF6 was degraded in vitro by active HtrA2 (mature HtrA2). The band of ATF6 was reduced significantly with the reaction of active HtrA2 with ATF6 for 4 hours (4h) or for overnight (O/N). On the other hand, the degradation of ATF6 by inactive HtrA2 (mature HtrA2 (S306A)) was not observed (Example 2).

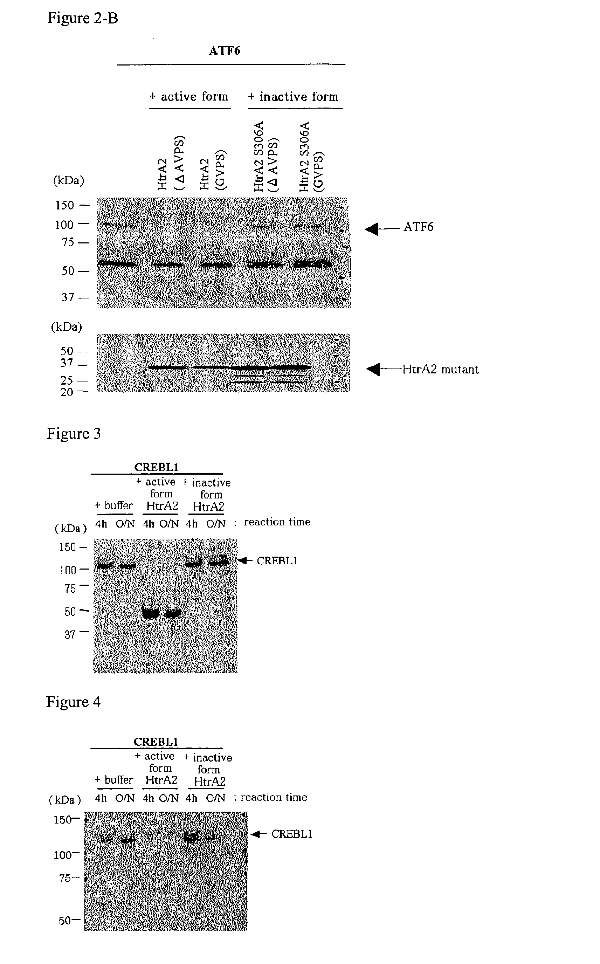

Fig. 2-A shows that CREBL1 was degraded in a cell by active HtrA2 mutant (mature HtrA2 (ΔAVPS) that lacks N-temiinal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with CREBL1, the band of CREBL1 was reduced significantly (upper panel). On the other hand, in the analysis using cells in which inactive HtrA2 mutant (the mature HtrA2 S306 (ΔAVPS) or the mature HtrA2 S306 (GVPS)) was co-expressed with CREBL1, reduction of the band of CREBL1 was not observed (upper panel). The expression of each HtrA2 mutant in the cells was almost same among the cells (lower panel) (Example 3).

Fig. 2-B shows that ATF6 was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine) (upper panel). In the analysis using cells in which the mature HtrA2 (ΔAVPS) or the mature HtrA2 (GVPS) was co-expressed with ATF6, the band of ATF6 was reduced significantly (upper panel). On the other hand, in the analysis using cells in which inactive HtrA2 mutant (the mature HtrA2 S306 (ΔAVPS) or the mature HtrA2 S306 (GVPS)) was co-expressed with ATF6, reduction of the band of ATF6 was not observed (upper panel). The expression of each HtrA2 mutant in the cells was almost same among the cells (lower panel) (Example 3)

Fig. 3 shows that the degradation of CREBL1 by active HtrA2 (mature HtrA2) was detected in the study using N-terminal tagged-CREBLl with biotinylated lysine residue within the protein and detecting the biotin. The band of biotinylated CREBL1 was reduced significantly with the reaction of active HtrA2 with CREBL1 for 4 hours (4h) or overnight (O/N), and the band around 50 kDa was detected which is considered to be a band indicating the degradation product of CREBL1 . On the other hand, the degradation of CREBL1 by inactive HtrA2 (mature HtrA2 (S306A)) was not observed, and the band around 50kDa as above was not detected (Example 4).

Fig. 4 shows that the degradation of CREBL1 by active HtrA2 (mature HtrA2) was detected in the study using N-terminal tagged-CREBL1 with biotinylated lysine residue within the protein by detecting the Tag ligated at the N-terminal. The band of N-temiinal tagged-CREBLI was reduced significantly with the reaction of active HtrA2 with CREBL1 for 4 hours (4h) or overnight (O/N). The band around 50 kDa that was detected in the study shown in Fig. 3, which is considered to be a band indicating the degradation product of CREBL1, was not detected. The detection was conducted using anti-Tag antibody. On the other hand, the degradation of CREBL1 by inactive HtrA2 (mature HtrA2 (S306A)) was not observed (Example 4).

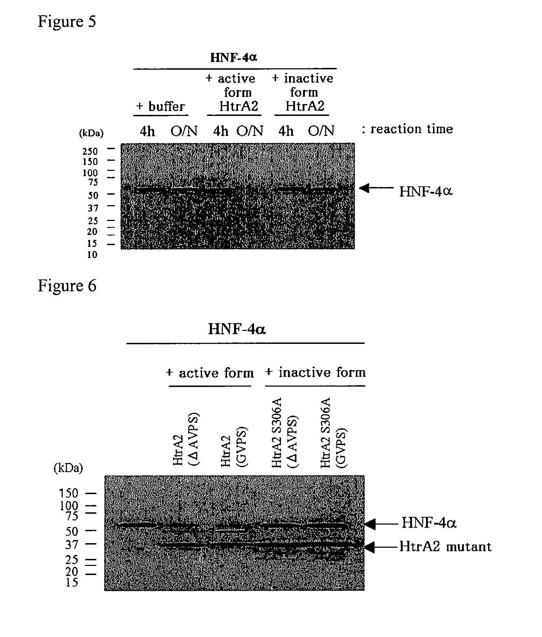

Fig. 5 shows that CREBL1 was degraded in vitro by active HtrA2 (mature HtrA2). The band of CREBL1 was significantly reduced with the reaction of active HtrA2 with CREBL1 for overnight (O/N). On the other hand, the degradation of CREBL1 by inactive HtrA2 (mature HtrA2 (S306A)) was not observed (Example 6).
Fig. 6 shows that CREBL1 was degraded in a cell by active HtrA2 mutant (mature HtrA2 (ΔAVPS) that lacks N-terminal four amino acid residues of the active HtrA2 (mature HtrA2); or mature HtrA2 (GVPS) with the substitution of alanine at the N-terminal amino acid of the active HtrA2 with glycine). In the analysis using cells in which the mature HtrA2 (ΔAVPS) or the mature HtrA2 (GVPS) was co-expressed with CREBL1, the band of CREBL1 was reduced significantly. On the other hand, in the analysis using cells in which inactive HtrA2 mutant (the mature HtrA2 S306 (ΔAVPS) or the mature HtrA2 S306 (GVPS)) was co-expressed with CREBL1, reduction of the band of CREBL1 was not observed (Example 7).

### DETAILED DESCRIPTION OF THE INVENTION

The present invention claims the benefit of priority from Japanese Patent Application No. 2003-342587, which is incorporated herein by reference in its entirety.

In the present specification, the term "polypeptide" may be used as a generic term which includes the followings: an isolated or a synthetic full-length protein; an isolated or a synthetic full-length polypeptide; and an isolated or a synthetic full-length oligopeptide. A protein, a polypeptide or an oligopeptide used herein comprises two or more amino acids that are bound to each other by peptide bond or modified peptide bond. Herein after, an amino acid may be represented by a single letter or by three letters.

In the present invention, the interaction of HtrA2 with CREBL1 and with HNF-4α was predicted *in-silico* according to the method described in the pamphlet of International Publication No. WO 01/67299. Further, it was revealed by way of experiment, for the first time, that an active form of HtrA2 degrades CREBL1, ATF6 that is a CREBL1 family protein and HNF-4α.

CREBL1 is one of ATF6 family and is also referred to as ATF6*β*. It has been known that locates in endoplasmic reticulum and the portion thereof translocates into nucleus upon endoplasmic reticulum stress after processing by S1P and S2P to work as a transcription factor. It is considered that both CREBL1 and ATF6 perceive the endoplasmic reticulum stress to induce transcription of chaperon genes, and involve in the recovery of the stress in cells and the survival of the cells (Non-Patent References 8 and 9). In addition, it has been reported that endoplasmic reticulum stress causes pancreatic *β* cell death, thereby to progress diabetes (Non-Patent Reference 7). From these facts, it is believed that the degradation of CREBL1 and/or ATF6 by HtrA2 causes the reduction or disappearance of the amount of these proteins, and thereby induces the enhanced cell death due to stress and the like, such as pancreatic *β* cell death, which results in the aggravation of diabetes.

Accordingly, to inhibit the degradation by HtrA2 of CREBL1 and/or ATF6 enables to inhibit the reduction or disappearance of the amount of at least one of these proteins, thereby enables to inhibit the cell death due to stress and the like, such as pancreatic *β* cell death. Further the prevention and/or treatment of diabetes can be conducted.

It has been clarified that loss of HNF-4α function causes disordered glucose metabolism and that HNF-4α gene is a causative gene for genetic type 2 diabetes MODY1 (Non-Patent References 10 and 11). From these facts, it is believed that the reduction or disappearance of the amount of HNF-4α causes disordered glucose metabolism resulting in aggravation of diabetes (for example, type 2 diabetes). In other words, it is believed that the degradation of HNF-4α by HtrA2 causes the reduction or disappearance of the amount of HNF-4α, thereby to give disordered glucose metabolism, resulting in aggravation of diabetes (for example, type 2 diabetes).

Therefore, to inhibit the degradation by HtrA2 of HNF-4α enables to inhibit the reduction or disappearance of the amount of HNF-4α, and as result, enables to normalize the glucose metabolism. Further, the prevention and/or treatment of diabetes, for example, type 2 diabetes can be conducted.

As described above, to inhibit the degradation by HtrA2 of at least one of CREBL1, ATF6 and HNF-4 and thereby to inhibit the reduction or disappearance of the amount of at least one of these proteins enable to achieve the prevention and/or treatment of diabetes. In comparison of the effect of the prevention and/or treatment of diabetes which is obtainable by inhibiting the degradation of any one of these proteins, the higher effect can be obtainable by inhibiting the degradation by HtrA2 of two or three of these proteins.

It is believed that inhibition of the degradation by HtrA2 of CREBL1 and/or ATF6 and inhibition of the degradation by HtrA2 of HNF-4α act in preventing and/or treating diabetes through their respective different mechanisms. The inhibition of the degradation by HtrA2 of CREBL1 and/or ATF6 is believed that it acts in preventing and/or treating diabetes through inhibition of pancreatic *β* cell death. In the mean while, the inhibition of the degradation by HtrA2 of HNF-4α is believed that it acts in preventing and/or treating diabetes (for example, type 2 diabetes), through normalization of glucose metabolism. Therefore, to inhibit both the degradation by HtrA2 of CREBL1 and/or ATF6 and the degradation by HtrA2 of HNF-4α enables to achieve the prevention and/or treatment of diabetes through both of their respective different mechanisms, thereby to provide a higher effect.

Based on these findings thus obtained, the present invention provides a method for inhibiting the degradation by HtrA2 of at least one of CREBL1, ATF6, and HNF-4α The present invention also provides a method for preventing and/or treating disorders attributable to the degradation of at least one of CREBL1, ATF6, and HNF-4αby HtrA2, such as diabetes.

A method for inhibiting the degradation of at least one of CREBL1, ATF6, and HNF-4α can be carried out by inhibiting the function of HTrA2.

The function of HtrA2 includes the protease activity to cleave at least one of CREBL1, ATF6, and HNF-4α. A protease generally cleaves one or more peptide bonds of a protein to take place the proteolysis. In fact, HtrA2 cleaved the peptide bonds of CREBL1 at the several sites to degrade CREBL1 (Example 4). It is then considered that similarly, HtrA2 cleaves the peptide bonds of ATF6 and HNF-4α at the one or more sites to degrade these proteins.

Therefore, a method for inhibiting the degradation of at least one of CREBL1, ATF6, and HNF-4α can be carried out by inhibiting the cleavage by HtrA2 of at least one of CREBL1, ATF6, and HNF-4. In the method for inhibiting the degradation, the degradation of CREBL1 is inhibited by inhibiting the cleavage by HtrA2 of CREBL1, the degradation of ATF6 is inhibited by inhibiting the cleavage by HtrA2 of ATF6, and the degradation of HNF-4α is inhibited by inhibiting the cleavage by HtrA2 of HNF-4α.

The function of HtrA2 also implies its interaction with at least one of CREBL1, ATF6, and HNF-4α "Interaction of HtrA2 with at least one of CREBL1, ATF6, and HNF-4α" as described herein means that HtrA2 and at least one of CREBL1, ATF6, and HNF-4α affect each other in a certain manner, and as a specifically result, HtrA2 may degrade at least one of CREBL1, ATF6, and HNF-4α. The term "in a certain manner" includes "by binding to", "by contacting with", or "by being adjacent to", and may be any manner as long as it allows their affection each other.

Therefore, the inhibition of HtrA2 from interacting with at least one of CREBL1, ATF6, and HNF-4α allows carrying out a method for inhibiting the degradation of at least one of CREBL1, ATF6, and HNF-4α.

A method for inhibiting the degradation of at least one of CREBL1, ATF6, and HNF-4α can be carried out by inhibiting the function of HtrA2 preferably in an in vitro sample comprising at least one of CREBL1, ATF6, and HNF-4α, and HtrA2.

The method for inhibiting the degradation of at least one of CREBL1, ATF6, and HNF-4α can be specifically carried out using a compound that inhibits the cleavage by HtrA2 of at least one of CREBL1, ATF6, and HNF-4α Such compound includes a compound that inhibits the protease activity of HtrA2, and preferably a compound that specifically inhibits the protease activity of HtrA2. The compound that specifically inhibits the protease activity of HtrA2 denotes such a compound that strongly inhibits the protease activity of HtrA2, but does not inhibit or weakly inhibits the activity of other enzymes. The compound that inhibits the protease activity of HtrA2 can be identified and obtained, for example, by adding a compound (hereinafter referred to as "test compound") in a system for detecting the degradation of substrate by HtrA2 using CREBL1, ATF6, or HNF-4α as the substrate, and then determining whether the test compound inhibits the degradation of the substrate. The compound that specifically inhibits the protease activity of HtrA2 can be obtained from the compounds identified as above by selecting a compound that inhibits more strongly the protease activity of HtrA2 than the activity of the other enzymes. The compound that inhibits the cleavage by HtrA2 of at least one of CREBL1, ATF6, and HNF-4α may be, for example, a polypeptide comprising the amino acid sequence of a site, in each of amino acid sequences of CREBL1, ATF6, HNF-4α or HtrA2, where HtrA2 interacts with CREBL1, ATF6, or HNF-4cx In particular, such a polypeptide derived from CREBL1, ATF6 or HNF-4α which serves as the substrate of HtrA2 can competitively inhibit the interaction of HtrA2 with CREBL1, ATF6 or HNF-4α, and can inhibit the cleavage by HtrA2 of these proteins. Such a polypeptide which further specifically inhibits the interaction of HtrA2 with CREBL1, ATF6, or HNF-4α is more preferably used in the method for inhibiting the degradation according to the present invention. The polypeptide that specifically inhibits the interaction of HtrA2 with at least one of CREBL1, ATF6 and HNF-4α, denotes a polypeptide that strongly inhibits the interaction of HtrA2 with at least one of CREBL1, ATF6, and HNF-4α, but does not inhibit or weakly inhibits the other protein-protein interaction. Further, the polypeptide comprising the amino acid sequence of a degradation site of CREBL1, ATF6, or HNF-4α by HtrA2 may be preferably a compound that inhibits the cleavage by HtrA2 of at least one of CREBL1, ATF6, and BNF-4α. Such a polypeptide which further specifically inhibits the degradation by HtrA2 of CREBL1, ATF6 or HNF-4α is preferably used in the method for inhibiting the degradation according to the present invention. The polypeptide that specifically inhibits the degradation by HtrA2 of CREBL1, ATF6 or HNF-4α, denotes such a polypeptide that strongly inhibits the degradation by HtrA2 of at least one of CREBL1, ATF6, and HNF-4α, but does not inhibit or weakly inhibits the degradation by HtrA2 of other proteins.

A method for inhibiting the degradation of at least one of CREBL1, ATF6, and HNF-4α can be carried out using a compound that inhibits the interaction of at least one of CREBL1, ATF6, and HNF-4α with HtrA2. The compound that inhibits the interaction of at least one of CREBL1, ATF6, and HNF-4α with HtrA2 which further shows the inhibitory effect specifically on the interaction is more preferably used in the method for inhibiting the degradation according to the present invention. The compound, which specifically inhibits the interaction of at least one of CREBL1, ATF6, and HNF-4α with HtrA2, denotes such a compound that strongly inhibits the interaction, but does not inhibit or weakly inhibits the other protein-protein interaction. Such a compound may be, for example, a polypeptide comprising the amino acid sequence of a site, in each of amino acid sequences of CREBL1, ATF6, HNF-4α or HtrA2, where CREBL1, ATF6, or HNF-4α interacts with HtrA2. In particular, such a polypeptide derived from CREBL1, ATF6 or HNF-4α which serves as the substrate of HtrA2 can competitively inhibit the interaction of CREBL1, ATF6 or HNF-4α, with HtrA2, and can inhibit the degradation by HtrA2 of these proteins.

A polypeptide that can be used in the method for inhibiting the degradation of at least one of CREBL1, ATF6, and HNF-4α, can be obtained by firstly designing polypeptides based on the amino acid sequence of CREBL1, ATF6, HNF-4α, or HtrA2, synthesizing them by peptide synthesis methods well-known in the art, and selecting a polypeptide that inhibits the degradation of at least one of CREBL1, ATF6, and HNF-4α by HtrA2. For example, the polypeptide comprising the amino acid sequence of an interaction site with HtrA2 or a degradation site by HtrA2 in each of amino acid sequences of CREBL1, ATF6, and HNF-4α may be preferably used. A polypeptide having an amino acid sequence derived from the thus specified polypeptide, in which a mutation such as a deletion, substitution, addition or insertion of one to several amino acids was introduced, is also included in the scope of the present invention. A polypeptide that also inhibits the degradation by HtrA2 of CREBL1, ATF6, or HNF-4α, is preferable for such a polypeptide into which the mutation is introduced. A polypeptide having the mutation may be a naturally existing polypeptide or a polypeptide in which a mutation was introduced. Techniques for introducing a mutation such as a deletion, substitution, addition or insertion are known. For example, the Ulmer technique (Non-patent Reference 12) may be utilized. When introducing a mutation as described above, in view of avoiding a change in the fundamental properties (such as physical properties, function, physiological activity, and immunological activity) of the polypeptide, mutual substitution among homologous amino acids (polar amino acids, non-polar amino acids, hydrophobic amino acids, hydrophilic amino acids, positive-charged amino acids, negative-charged amino acids and aromatic amino acids or the like) may be readily conceived. Furthermore, these usable polypeptides can be modified to the extent that no significant functional change is involved, such as modification of its constituent amino group or carboxyl group and the like by an amidation and the like.

The aforementioned polypeptide can be produced by common methods that are known in peptide chemistry. A method described in the References (Non-patent documents 13 and 14), for example, may be used, although the methods are not limited thereto, and known methods can be broadly utilized. Particularly, a peptide synthesis method using an ordinary liquid phase method and solid phase method, such as, for example, the Fmoc method can be used. Moreover, an amino acid synthesizer that is commercially available can be used for producing the polypeptide. Alternatively, a technology of gene manipulation can be also used for producing the polypeptide. For example, a gene encoding a polypeptide of interest can be used to prepare a recombinant expression vector that can express the gene in a host cell, followed by transfection of the recombinant expression vector into a suitable host cell such as E. coli to obtain a transformant. Culturing the transformant and collecting the polypeptide of interest from the obtained culture product achieves the production of the polypeptide.

A method for inhibiting the degradation of at least one of CREBL1, ATF6, and HNF-4α can be also carried out using an antibody that recognizes CREBL1, ATF6, HNF-4α or HtrA2 and inhibits the degradation by HtrA2 of CREBL1, ATF6, or HNF-4α Such an antibody can be produced by known methods for preparing an antibody using CREBL1, ATF6, HNF-4α a polypeptide comprising the amino acid sequence of an interaction site of CREBL1, ATF6, or HNF-4α with HtrA2, or the like as an antigen.

A compound that inhibits the cleavage by HtrA2 of at least one of CREBL1, ATF6, and HNF-4α can be obtained by utilizing a known pharmaceutical screening system using the degradation by of at least one of CREBL1, ATF6, and HNF-4α by HtrA2 as an indicator. Since the inhibition of the cleavage by HtrA2 of at least one of CREBL1, ATF6, and HNF-4α results in the inhibition of the degradation of these proteins, it is believed that a compound that inhibits the cleavage of these proteins can inhibits the degradation of these proteins. Therefore, a compound that inhibits the degradation by HtrA2 of at least one of CREBL1, ATF6, and HNF-4α can be also obtained by a method of identifying a compound that inhibits the cleavage by HtrA2 of at least one of CREBL1, ATF6, and HNF-4α The compound that inhibits the cleavage and degradation by HtrA2 of at least one of CREBL1, ATF6, and HNF-4α can be identified, for example, by selecting conditions that allow for the cleavage and degradation by HtrA2 of at least one of CREBL1, ATF6, and HNF-4α, contacting a compound to be tested (hereinafter, referred to the test compound) with HtrA2 and/or at least one of CREBL1, ATF6 and HNF-4α under the conditions selected; employing a system that uses a signal and/or a marker capable of detecting the cleavage and degradation of at least one of CREBL1, ATF6, and HNF-4α, and then detecting the presence, the absence or the change of the signal and/or the marker. The condition for allowing the cleavage and degradation by HtrA2 at least one of CREBL1, ATF6, and HNF-4α may be a condition in vitro or in vivo. The method of identifying a compound can be carried out, for example, by using a cell in which HtrA2 is co-expressed with at least one of CREBL1, ATF6, and HNF-4α. The co-expression in the cell can be accomplished by using an appropriate vector containing a polynucleotide encoding CREBL1, ATF6 or HNF-4α and an appropriate vector containing a polynucleotide encoding HtrA2, and transfecting them into the cell by a conventional technology of gene manipulation. The vector containing a polynucleotide encoding CREBL1, ATF6, HNF-4α or HtrA2 can be used solely or in combination thereof to transfect the cell. For example, the cell may be a eukaryotic cell, preferably an animal cell, more preferably a cultured animal cell line, further preferably a cultured mammal cell line. Contact of the test compound with HtrA2 and/or at least one of CREBLI, ATF6, and HNF-4α may be conducted prior to a cleavage reaction or degradation reaction of at least one of CREBL1, ATF6, and HNF-4αr by HtrA2, or may be conducted by allowing the test compound to coexist in the cleavage reaction or degradation reaction. As used herein, the term "signal" means chemicals that can be detected directly based on its physical properties or chemical properties. The term "marker" means chemicals that can not be detected itself by physical properties or chemical propertied, but can generate the aforementioned signal via a chemical reaction and can be detected indirectly by using the physical properties or chemical properties of the generated signal as an indicator. As a signal and/or marker, the followings may be used: a reporter gene (such as chloramphenicol acetyl transferase gene), radioisotope, tag peptides (such as His-tag, Myc-tag, HA-tag, FLAG-tag or Xpress-tag), enzymes such as GST, biotin and fluorescent protein. The signal and/or marker are not limited to these chemicals, and any labeling chemicals generally used in a method of identifying compounds may be utilized. Methods for detecting these signals or markers are known to those skilled in the art. As a convenient method, the cleavage and degradation of at least one of CREBL1, ATF6, and HNF-4αby HtrA2 can be detected by determining the presence, the absence and/or the change of the amount of these proteins or the amount of degradation products of these proteins. Determination of the amount of these proteins or the amount of degradation products of these proteins can be carried out using a known method for detecting a protein or peptide, such as, for example, Western blotting.

A compound that inhibits the interaction of HtrA2 with at least one of CREBL1, ATF6, and HNF-4α can be obtained by aforementioned identification method.

HtrA2, CREBL1, and ATF6 are known proteins and disclosed in GenBank with the accession numbers NM_013247, NM_00438, and NM_007348, respectively. Further, HNF-4α is also a known protein and disclosed in the Swiss-Prot database with the accession number P41235 (registered gene name is HNF4A).

The amino acid sequence of HtrA2 used in the Examples is shown in SEQ ID NO: 4. The nucleotide sequence of HtrA2 DNA encoding the amino acid sequence set forth in SEQ ID NO: 4 is shown in SEQ ID NO: 3. The polypeptide shown by the amino acid sequence set forth in SEQ ID NO: 4 is a mature HtrA2. The mature HtrA2 denotes a mature protein that is generated from HtrA2 precursor protein (SEQ ID NO: 2) by cleavage of its N-terminal 133 amino acid residues and has a protease activity. Hereinafter, the HtrA2 with protease activity may be referred to as an active HtrA2. Further, a protein (SEQ ID NO: 8, which may be referred to as mature HtrA2 (ΔAVPS)) that lacks the N-temlinal four amino acid residues (AVPS) in the mature HtrA2, or a protein (SEQ ID NO: 10, in some cases referred to as mature HtrA2 (GVPS)) with the substitution of alanine among the N-terminal four amino acid residues of the mature HtrA2 with glycine may be used as active HtrA2. Such HtrA2 with an introduced mutation can be used as active HtrA2 since there is no change in the protease activity. In the meantime, HtrA2 without protease activity may be in some cases referred to as inactive HtrA2. The inactive HtrA2 may be, for example, HtrA2 mutant without protease activity resulting from a mutation of the amino acid residue that is present at the site necessary for protease activity of HtrA2 in the amino acid sequence of HtrA2. The site necessary for protease activity of HtrA2 has a protease activity domain, more preferably the 174^{th} serine residue of mature HtrA2 (SEQ ID NO: 4) (which is corresponding to the 306^{th} residue of the precursor protein (SEQ ID NO: 2)). More specifically, the inactive HtrA2 may be, for example, HtrA2 mutant (SEQ ID NO: 6, which may be referred to as mature HtrA2 (S306A)) with a substitution of the 174^{th} serine residue of mature HtrA2 (which is corresponding to the 306^{th} residue of the precursor protein (SEQ ID NO: 2)) with alanine. Further, a protein (SEQ ID NO: 12, in some cases referred to as mature HtrA2 (S306A, ΔAVPS)) that lacks the N-terminal four amino acid residues (AVPS) in the mature HtrA2 (S306A), or a protein (SEQ ID NO: 14, in some cases referred to as mature HtrA2 (S306A, GVPS)) with the substitution of alanine among the N-terminal amino four amino acid residues of the mature HtrA2 with glycine may be used as inactive HtrA2.

The amino acid sequence of CREBL1 used in the Examples is shown in SEQ ID NO: 16. Furthermore, the nucleotide sequence of CREBL1 DNA encoding the amino acid sequence set forth in SEQ ID NO: 16 is shown in SEQ ID NO: 15. It was found that the nucleotide sequence set forth in SEQ ID NO: 15 had a difference in one nucleotide compared with the known nucleotide sequence of CREBL1 with accession number NM_00438 (see Example 2).

The amino acid sequence of ATF6 used in the Examples is shown in SEQ ID NO: 18. In addition, the nucleotide sequence of ATF6 DNA encoding the amino acid sequence set forth in SEQ ID NO: 18 is shown in SEQ ID NO: 17. It was found that the nucleotide sequence set forth in SEQ ID NO: 17 had a difference in fifteen nucleotides compared with the known nucleotide sequence of ATF6 with accession number NM_007348 (see Example 2).

The amino acid sequence of HNF-4α used in the Examples is shown in SEQ ID NO: 35. Further, the nucleotide sequence of HNF-4α DNA encoding the amino acid sequence set forth in SEQ ID NO: 35 is shown in SEQ ID NO: 34. The amino acid sequence set forth in SEQ ID NO: 35 was identical with the known amino acid sequence of HNF-4α with accession number P41235 (registered gene name is HNF4A) of Swiss-Prot database (see Example 6).

In the present invention, HtrA2, CREBL1, ATF6 and HNF-4α, as well as each gene that encodes each of these proteins, are not limited to proteins or genes that are shown by the above-exemplified amino acid sequences or the above-exemplified nucleotide sequences, and include generally reported HtrA2, CREBL1, ATF6 and HNF-4α

HtrA2, CREBL1, ATF6 and HNF-4α used in the present invention can be those prepared from cells in which these are expressed by the technology of gene manipulation, or from any biological samples, or can be the products of cell-free synthesis systems or the chemical synthesis products.
These can be subsequently further purified for use. Further, cells in which one or more of HtrA2, CREBL1, ATF6 and HNF-4α are expressed by the technology of gene manipulation can be used. Moreover, HtrA2, CREBL1, ATF6 and HNF-4α can be labeled by ligating a different type of protein or polypeptide thereto at the N-terminus or the C-terminus, directly or indirectly via a linker peptide and the like, by means of, for example, the technology of gene manipulation, as long as it has no influence upon the interaction of HtrA2 with CREBL1, ATF6 or HNF-4α, and upon the function of these proteins, such as protease activity of HtrA2, and a feature of CREBL1, ATF6 and HNF-4α as an enzyme substrate. As a different type of protein or polypeptide, β-galactosidase, an immunoglobulin Fc fragment (such as IgG), and a tag peptide (such as His-tag, Myc-tag, HA-tag, FLAG-tag, or Xpress-tag) may be used.

A polynucleotide encoding HtrA2, CREBL1, ATF6 or HNF-4α can be prepared from a human cDNA library by the technology of gene manipulation. A vector containing the polynucleotide that encodes HtrA2, CREBL1, ATF6 or HNF-4α can be obtained by introducing the polynucleotide using the technology of gene manipulation into a suitable expression vector, such as a vector derived from a bacterial plasmid.

A test compound may be, for example, a compound derived from a chemical library or natural products, as well as a compound obtained by drug design based on the primary structure or tertiary structure of HtrA2, CREBL1, ATF6 or HNF-4α. Alternatively, a compound obtained by drug design based on the structure of a polypeptide that comprises an amino acid sequence of an interaction site with HtrA2 or of a cleavage site by HtrA2 in the amino acid sequence of at least one selected from the group consisting of CREBL1, ATF6 and HNF-4α is also suitable as a test compound.

The method of identifying a compound according to the present invention can be specifically carried out by adding a test compound to the in vitro protease assay (Examples 2 and 6) using, for example, HtrA2 and at least one of CREBL1, ATF6, and HNF-4a Mature HtrA2 of an active type can be prepared from E-coli transfected with an appropriate vector containing a mature HtrA2 DNA obtained from the human kidney cDNA library by the technology of gene manipulation. CREBL1 can be prepared from a cultured animal cell line (for example, HEK293T cell) transfected with an appropriate vector containing CREBL1 DNA obtained from the human brain cDNA library by the technology of gene manipulation. ATF6 can be prepared from an animal cell (for example, HEK293T cell) transfected with an appropriate vector containing ATF6 DNA obtained from the human mammary gland cDNA library by the technology of gene manipulation. HNF-4α can be prepared from a cultured animal cell line (for example, HEK293T cell) transfected with an appropriate vector containing HNF-4a DNA obtained from the human brain polyA⁺RNA by the technology of gene manipulation. CREBL1, ATF6, HNF-4α, and HtrA2 are contained in the soluble fractions of lysates of the cells in which these proteins are respectively expressed. CREBL1, ATF6, and HNF-4α are preferably expressed as a protein ligated with a tag peptide at the N-terminal or C-terminal, and are favorably purified for use by means of immuno-precipitation using an antibody against the tag peptide. For example, these proteins are subjected to immuno-precipitation using an antibody against the tag peptide, and then captured on a resin (such as protein G sepharose) for use. The in vitro protease assay can be carried out by adding mature HtrA2 to CREBL1*, ATF6 or HNF-4α captured on a resin, reacting them each other at 37°C overnight, and detecting these proteins by western blotting. With regard to ATF6, the protease assay may be performed with 4 hours reaction, since ATF6 is degraded by mature HtrA2 in the reaction at 37°C for 4 hours. Detection by western blotting can be performed using an antibody against a tag peptide ligated to CREBL1, ATF6 or HNF-4α Since CREBL1, ATF6 or HNF-4α is degraded by HtrA2, the amount of these proteins detected by western blotting is reduced or disappeared compared with a case without adding HtrA2. In the case that the reduction or disappearance of the amount of CREBL1, ATF6 or HNF-4α is inhibited by adding a compound to the reaction of mature HtrA2 with CREBL1, ATF6 or HNF-4α in comparison with a case without adding a compound, it can be judged that the test compound inhibits the degradation by HtrA2 of CREBL1, ATF6 or HNF-4α The test compound may be previously contacted with CREBL1, ATF6 or HNF-4α, or mature HtrA2, and then the reaction of the mature HtrA2 with CREBL1, ATF6 or HNF-4α may be conducted.

Further, the method of identifying a compound according to the present invention can be specifically carried out by adding a test compound to the intracellular protease assay (Examples 3 and 7) using, for example, such a cell in which at least one of CREBL1, ATF6, and HNF-4α are co-expressed with HtrA2. Co-expression of at least one of CREBL1, ATF6 and HNF-4α, with HtrA2 can be performed by transfecting a cultured animal cell line (for example, HEK293T cell) with an appropriate vector containing each of CREBL1 DNA, ATF6 DNA and HNF-4α DNA prepared as mentioned above and an appropriate vector containing HtrA2 DNA by the technology of gene manipulation. CREBL1, ATF6 and HNF-4α are preferably expressed as the proteins having tag peptides ligated to the N-terminal or C-terminal of these proteins, in order to detect the proteins easily. Active HtrA2 is used for HtrA2. The active HtrA2 suitably used are mature HtrA2, preferably mature HtrA2 (ΔAVPS) that lacks N-terminal four amino acid residues (AVPS) of mature HtrA2, or mature HtrA2 (GVPS) that has a substitution of alanine among the N-terminal four amino acid residues with glycine. It has been reported that N-terminal four amino acid residues (AVPS) of mature HtrA2, which is a binding motif to IAPs (Inhibitor of apoptosis proteins) family protein acting for inhibiting cell death, bind to IAPs to inhibit their action, thereby to accelerate caspase dependent cell death. Since this action may be likely to give an influence upon the protease assay in a cell, it is preferable to use mature type HrtA2 (ΔAVPS) or mature HtrA2 (GVPS) which does not bind to IAPs. The intracellular protease assay can be performed by culturing a cell in which at least one of CREBL1, ATF6, and HNF-4α are co-expressed with HtrA2 at 37°C for 48 hours, together with a test compound, and then lysing the cell to detect CREBL1, ATF6 or HNF-4α containing in the cell lysate by western blotting. Detection by western blotting can be carried out using an antibody against the tag peptide ligated to CREBL1, ATF6, or HNF-4α Since CREBL1, ATF6 or HNF-4α is degraded by HtrA2, the amount of these proteins detected by western blotting is reduced or disappeared compared with a case without adding HtrA2. In the case that the reduction or disappearance of the amount of CREBL1, ATF6 or HNF-4α is inhibited by adding a compound in comparison with a case without adding a compound, it can be judged that the test compound inhibits the degradation by HtrA2 of CREBL1, ATF6 or HNF-4α

The method of identifying a compound according to the present invention is not limited to the specific examples such as the aforementioned in vitro protease assay or the intracellular protease assay. An identification method well known for a pharmaceutical screening may be used.

A compound obtained by the method of identifying a compound according to the present invention can be utilized as an agent for inhibiting the degradation by HtrA2 of at least one of CREBL1, ATF6, and HNF-4α The compound can be selected in consideration of a balance between the biological usefulness and toxicity to prepare a pharmaceutical composition. In preparation of the pharmaceutical composition, the compound may be used alone or in combination.

The compound that inhibits the degradation by HtrA2 of at least one of CREBL1, ATF6, and HNF-4α can be used as an active ingredient for an agent for preventing and/or treating diabetes. In other words, an agent for preventive and/or treating diabetes can be prepared using one or more compounds that inhibit the degradation by HtrA2 of at least one of CREBL1, ATF6, and HNF-4α. Such compounds can be obtained by the aforementioned method of identifying a compound. It is believed that a compound that inhibits the degradation by HtrA2 of CREBL1 and/or ATF6 can inhibit cell death (for example, pancreatic *β* cell death) due to excessive endoplasmic reticulum stress, and thereby to exert effects for the prevention and/or treatment of diabetes. Further, it is believed that a compound that inhibits the degradation by HtrA2 of HNF-4α can normalize the glucose metabolism, and thereby to exert effects for the prevention and/or treatment of diabetes (for example, type 2 diabetes). Thus, it is believed that a compound that inhibits the degradation by HtrA2 of CREBL1 and/or ATF6 and a compound that inhibits the degradation by HtrA2 of HNF-4α act in preventing and/or treating diabetes through their respective different mechanisms. Therefore, an agent for preventing and/or treating diabetes, which contains a compound that inhibits the degradation by HtrA2 of CREBL1 and/or ATF6 in combination with a compound that inhibits the degradation by HtrA2 of HNF-4α, is more preferable, since it acts in preventing and/or treating diabetes through both of respective different mechanisms, and thereby provides a higher efficacy compared with an agent for preventing and/or treating diabetes which contains either of these compounds.

The method for preventing and/or treating diabetes according to the present invention can be carried out by inhibiting the degradation byHtrA2of at least one of CREBL1, ATF6, and HNF-4α It is believed that to inhibit the degradation by HtrA2 of CREBL1 and/or ATF6 enables to inhibit cell death (for example, pancreatic *β* cell death) due to excessive endoplasmic reticulum stress, thereby to exert effects in preventing and/or treating diabetes. Further, it is believed that to inhibit the degradation by HtrA2 of HNF-4α enables to normalize the glucose metabolism, thereby to exert effects in preventing and/or treating diabetes (for example, type 2 diabetes). It is believed that inhibition of the degradation by HtrA2 of CREBL1 and/or ATF6 and inhibition of the degradation by HtrA2 ofHNF-4α act in preventing and/or treating diabetes through their respective different mechanisms. Therefore, a method for preventing and/or treating diabetes, which comprises inhibiting the degradation by HtrA2 of CREBL1 and/or ATF6 as well as inhibiting the degradation by HtrA2 of HNF-4α, is more preferable, since it acts in preventing and/or treating diabetes through both of respective different mechanisms, and thereby provides a higher efficacy compared with a method for preventing and/or treating diabetes comprising inhibiting either of the degradation of these proteins.

The method for preventing and/or treating diabetes according to the present invention can be carried out using the aforementioned compound that inhibits the degradation by HtrA2 of at least one of CREBL1, ATF6, and HNF-4α. Further, the method for preventing and/or treating diabetes according to the present invention can be carried out using the aforementioned method for inhibiting the degradation of at least one of CREBL1, ATF6, and HNF-4α.

Since it is considered that both CREBL1 and ATF6 are involved in the recovery from the endoplasmic reticulum stress and survival of a cell (Non-Patent References 8 and 9), a method for inhibiting cell death can be carried out by inhibiting the degradation by HtrA2 of CREBL1 and/or ATF6. Further, the compound that inhibits the degradation by HtrA2 of CREBL1 and/or ATF6 can be used as an inhibitor of cell death. Namely, the inhibitor of cell death can be prepared using one or more compounds that inhibit the degradation by HtrA2 of CREBL1 and/or ATF6. Further, since it is reported that pancreatic *β* cell death is caused by the endoplasmic reticulum stress (Non-Patent Reference 7), the method and the agent for inhibiting cell death according to the present invention can be preferably used for a method and an agent for inhibiting pancreatic *β* cell death. More preferably, these can be used for a method and an agent for inhibiting t pancreatic *β* cell death due to endoplasmic reticulum stress.

The method for inhibiting cell death according to the present invention can be carried out using a method for inhibiting the degradation by HtrA2 of CREBL1 and/or ATF6. The compound that inhibits the degradation by HtrA2 of CREBL1 and/or ATF6 can be obtained by the aforementioned method of identifying a compound. Moreover, the method for preventing and/or treating diabetes can be carried out using the method and the agent for inhibiting cell death. The method for inhibiting cell death can be preferably carried out in an in vitro sample containing at least CREBL1 and/or ATF6 together with HtrA2, by inhibiting the degradation by HtrA2 of CREBL1 and/or ATF6.

Since it has been clarified that loss of HNF-4α function causes disordered glucose metabolism and that HNF-4α gene is a causative gene for genetic type 2 diabetes MODY1 (Non-Patent References 10 and 11), a method for preventing and/or treating type 2 diabetes can carried out by inhibiting the degradation by HtrA2 of HNF-4α Besides, a compound that inhibits the degradation by HtrA2 of HNF-4α can be used as an active ingredient in an agent for preventing and/or treating type 2 diabetes. In other words, an agent for preventing and/or treating type 2 diabetes can be prepared using one or more compounds that inhibit the degradation by HtrA2 of HNF-4α.

The method for preventing and/or treating type 2 diabetes according to the present invention can be carried out using a method for inhibiting the degradation by HtrA2 of HNF-4α. The compound that inhibits the degradation by HtrA2 of HNF-4α can be obtained by the aforementioned method of identifying a compound.

The agent for preventing and/or treating diabetes mellitus according to the present invention can be a pharmaceutical agent prepared by using one or more of the aforementioned compounds so as to contain the effective amount thereof. Generally, the pharmaceutical agent is preferably prepared as a pharmaceutical composition that includes one or more kinds of a pharmaceutical carrier.

An amount of the effective ingredient containing in the pharmaceutical preparation can be suitably selected from wide range. In general, a suitable amount may fall within a range of approximately 0.00001 to 70 wt%, preferably approximately 0.0001 to 5 wt%.

A pharmaceutical carrier may be a filler, extender, binder, wetting agent, disintegrator, lubricant, diluent and excipient that are generally used in accordance with the form of use of the formulation. These can be suitably selected and used in accordance with the administration form of the preparation to be obtained.

More concretely, the pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these may be suitably selected and used in accordance with the dosage form of the pharmaceutical composition.

As desired, various components that may be used in conventional protein preparation, such as a stabilizer, bacteriocide, buffer agent, isotonizing agent, chelating agent, surfactant, or pH adjuster, may be suitably contained in the pharmaceutical composition.

As a stabilizer, the followings may be used: human serum albumin, common L-amino acids, sugars and cellulose derivatives. These can be used independently or in combination with a surfactant and the like. Especially, use of these in such a combination may give increased stability of an effective ingredient. An L-amino acid is not particularly limited, and may be any one of glycine, cysteine, glutamic acid and the like. A sugar is not particularly limited, and may be any one of monosaccharides (such as glucose, mannose, galactose and fructose), sugar alcohols (such as mannitol, inositol and xylitol), disaccharides (such as sucrose, maltose and lactose), polysaccharides (dextran, hydroxypropylstarch, chondroitin sulfate and hyaluronic acid), derivatives thereof and so on. A cellulose derivative is not particularly limited, and may be any one of methylcellulose, ethylcellulose, hydroxyethylcellulose, hydroxypropylcellulose, hydroxypropyhnethlcellulose, sodium carboxymethylcellulose and the like.

A surfactant is not particularly limited, and can be both an ionic surfactant and a non-ionic surfactant. As a surfactant, the followings may be used: polyoxyethyleneglycol sorbitan alkyl ester system, polyoxyethylene alkyl ether system, sorbitan monoacyl ester system, and atty acid glyceride system.

As a buffer agent, the followings may be used: boric acid, phosphoric acid, acetic acid citric acid, ε-aminocaproic acid, glutamic acid and/or a salt thereof D for example, an alkaline metal salt and an alkali earth metal salt, such as sodium salt, kalium salt, calcium salt and magnesium salt□.

As an isotonizing agent, the followings may be used: sodium chloride, kalium chloride, sugars and glycerin.

As a chelating agent, sodium edentate and citric acid may be used.

The pharmaceutical agent and pharmaceutical composition according to the present invention can be used as a solution preparation. Alternatively, they can be freeze-dried so as to be in good state in preservation and can be used by dissolving them in water, a buffered solution containing saline and the like, and so on and then adjusting to suitable concentration at the time of using.

Suitable dosage ranges of the pharmaceutical composition are not particularly limited, and can be determined according to the following: effectiveness of the ingredients contained therein; the administration form; the route of administration; the type of disease; the characteristics of the subject (e.g., body weight, age, symptomatic conditions and whether being taking other pharmaceutical agent) and the judgment of the physician in charge. In general, a suitable dosage may fall, for example, within a range of about 0.01 *µ*g to 100 mg per 1 kg of the body weight of the subject, and preferably within a range of about 0.1 *µ*g to 1 mg per 1 kg. However, a dosage may be altered using conventional experiments for optimization of a dosage that are well known in the art. The aforementioned dosage can be divided for administration once to several times a day. Alternatively, periodic administration once every few days or few weeks can be employed.

When administering the pharmaceutical composition of the present invention, the pharmaceutical composition may be used alone or may be used together with other compounds or pharmaceutical agents necessary for the treatment.

In terms of a route of administration, it may be either systemic administration or local administration. The route of administration that is appropriate for a particular disease, symptomatic conditions, or other factors should be selected. For example, parenteral administration including normal intravenous injection, intraarterial administration, subcutaneous administration, intracutaneous administration and intramuscular administration can be employed. Oral administration can be also employed. Further, transmucosal administration or dermal administration can be employed.

In terms of an administration form, various forms can be selected in accordance with a treatment purpose. Typically, for example, an administration form of solid formulation may be used, such as a tablet, a pill, powder, powdered drug, fine granule, granule or a capsule, as well as an administration form of liquid formulation such as an aqueous formulation, ethanol formulation, suspension, fat emulsion, liposome formulation, clathrate such as cyclodextrin, syrup and an elixir. These can be further classified according to the administration route into oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation and the like, which can be respectively blended, formed and prepared according to conventional methods.

The present invention further provides a reagent kit including at least one member selected from the group consisting of HtrA2, a polynucleotide encoding HtrA2, and a vector containing a polynucleotide encoding HtrA2; and at least one member selected from the group consisting of CREBL1, ATF6 and HNF-4α, a polynucleotide encoding CREBL1, ATF6, or HNF-4α, and a vector containing the polynucleotide encoding CREBL1, ATF6, or HNF-4α. The reagent kit can be used, for example, in the identification method for the present invention.

The aforementioned reagent kit may include chemicals that are necessary for carrying out a determination, such as a signal and/or a marker for detecting the degradation of at least one member selected from a group consisting of CREBL1, ATF6 and HNF-4α by HtrA2, buffer solutions and salts. The reagent kit may also include chemicals such as stabilizers and/or antiseptic agents. At the time of preparation, methods for preparation may be introduced in accordance with the respective chemicals to be used.

Hereinafter, the present invention may be explained more particularly with examples; however, the present invention is not limited to the following examples.

### Example 1

### (In-silico search for proteins having a function to interact with HtrA2)

The prediction of proteins that have a function to interact with HtrA2 was conducted according to the method described in the pamphlet of International Publication No. WO 01/67299. Concretely, the amino acid sequence of HtrA2 was decomposed into oligopeptides having a predetermined length in order to search in a database for proteins having the amino acid sequence of each of the oligopeptides, or having homologous amino acid sequences to these amino acid sequences. Then, local alignment was conducted between the proteins obtained and HtrA2 to identify proteins having a high local alignment score that might be capable of interacting with HtrA2.

As a result of analysis, CREBL1 was identified as a protein being predicted to have a function to interact with HtrA2.

### Example 2

### (In vitro protease assay)

In order to demonstrate experimentally the interaction of CREBL1 or ATF6 that is a family thereof with HtrA2, in vitro protease assay was performed.

### <Materials and their preparation>

In this Example, mature HtrA2 (SEQ ID NO: 4) and mature HtrA2 (S306A) (SEQ ID NO: 6) to each of which histidine (His)-tag was ligated at the C-terminal were used as active HtrA2 and inactive HtrA2, respectively. Further, CREBL1 (SEQ ID NO: 16) and ATF6 (SEQ ID NO: 18) to each of which c-Myc-tag was ligated at the N-terminal were used as a protein that was predicted to interact with HtrA2.

### 1. Cloning of mature HtrA2 and mature HtrA2 (S306A), and preparation of expression plasmid

In order to obtain a gene encoding mature HtrA2 (a portion of precursor HtrA2 (DNA nucleotide sequence thereof and amino acid sequence encoded by the DNA are shown in SEQ ID NO: 1 and SEQ ID NO: 2) from 134^{th} to 458^{th} amino acid residues), mature HtrA2 gene was amplified by polymerase chain reaction (PCR) and cloned into pCR-BluntII-TOPO vector (Invitrogen). The PCR was carried out by using Human Kidney QUICK-Clone cDNA (Clontech) as a template, HtrA2-F1 primer (with NdeI site and ATG at the 5' side, SEQ ID NO: 19), HtrA2-RS primer (with XhoI site instead of termination codon, SEQ ID NO: 20), and KOD-plus (Toyobo) as DNA polymerase. The nucleotide sequence was determined by sequencer (Applied Biosystems /Hitachi: ABI3100). The nucleotide sequence of mature HtrA2 DNA obtained in this example, and amino acid sequence encoded by the DNA are shown in SEQ ID NO: 3 and SEQ ID NO: 4, respectively. E-coli expression plasmid for mature HtrA2 was obtained by digesting the cloned mature HtrA2 gene with NdeI and XhoI, and then integrating it into pET24b vector (Novagen.

It has been reported that a mutant (mature HtrA2 (S306A)) with a substitution of 174^{th} serine of mature HtrA2 (which is corresponding to 306^{th} in precursor protein (SEQ ID NO: 2)) with alanine does not exhibit protease activity. Then, E-coli expression plasmid for mature HtrA2 (S306A) that is of inactive type was prepared using mature HtrA2 expression plasmid as a template, with QuickChange XL Site-Directed Mutagenesis kit (Stratagene) using HtrA2-MF primer (SEQ ID NO: 21) and HtrA2-MR primer (SEQ ID NO: 22). The nucleotide sequence was determined by sequencer. The nucleotide sequence of mature HtrA2 (S306A) DNA obtained in this example and amino acid sequence encoded by the DNA are shown in SEQ ID NO: 5 and SEQ ID NO: 6, respectively.

### 2. Expression and purification of mature HtrA2 and mature HtrA2 (S306A)

E-coli expression plasmid for HtrA2 was transfected into E-coli BL21 Star (DE3) competent cell (Invitrogen) to obtain a transformant The transformant was incubated in LB culture medium (100 ml) at 26°C, and expression induction of mature HtrA2 protein was carried out by adding isopropyl-1-thio-β-D-galactopyranoside (IPTG) to the final concentration of 0.1 mM when absorbance (OD) reached in a range of 0.68 to 0.81. After further incubation for overnight, E-coli that expressed mature HrtA2 was separated by centrifugation and collected. E-coli thus obtained was suspended in lysis buffer A (Lysis buffer: phosphate buffered saline (PBS)/1% TritonX-100, 1*µ*g/ml pepstatin, 5*µ*M E64) followed by treatment with sonicator (15 sec x 10 times) under ice-cooling to disrupt the fungus body. The disrupted fungus body solution was subjected to centrifugation (15,000 rpm, 30 min, 4°C) to separate into a soluble fraction (supernatant) and an insoluble fraction. Soluble fraction containing mature HtrA2 was added to ProBondresin (Invitrogen: 1 ml prepacked) that had been pre-equilibrated with 1% Triton X-100 in phosphate buffered saline (PBS), and mixed at 4°C for 30 min, and then washed three times with washing buffer (20 mM sodium phosphate (pH 6.0), 500 mM NaCl). Mature HtrA2 adsorbed by the resin was eluted in stepwise by elution buffer (20 mM, sodium phosphate (pH 6.0) 500 mM NaCI) each containing 50,100,200,350,500 mM imidazole. Each eluate was subjected to SDS-PASGE to identify the fi-action containing mature HtrA2. As a result, mature HtrA2 was eluted by 350 - 500 mM imidazole. The fraction containing mature HtrA2 was dialyzed against 150 mM NaCl, 50 mM Tris-HCl (pH 7.5), followed by centrifugation to remove impurities. The resultant supernatant was concentrated followed by subjecting to the quantitative measurement of protein by Coomassie Plus Protein Assay (Pierce) while bovine serum albumin (BSA) was used as the standard protein. Similarly, expression and purification of mature HtrA2 (S306A) were performed.

### 3. Cloning of CREBL1 and ATF6, and preparation of expression plasmid

CREBL1 gene was amplified by PCR and cloned into pCMV-Tag 5. The PCR was carried out using Human Brain cDNA as a template, 83-F primer (with EcoRI site and GCC immediately before ATG, SEQ ID NO: 23), 83-R primer (with XhoI site instead of the termination codon, SEQ ID NO: 24), and KOD-plus as DNA polymerase.

Then, CREBL1 gene was amplified by PCR and cloned into pCR-BluntII-TOPO vector. The PCR was carried out using the CREBL1 gene cloned into pCMV-Tag 5 as a template, ATF6-NF1 primer (with BamHI site instead of ATG, SEQ ID NO: 25), ATF6-NR1 primer (with XhoI site following to termination codon, SEQ ID NO: 26), and KOD-plus as DNA polymerase. The nucleotide sequence was determined by the sequencer. It was found that the nucleotide sequence obtained in this example had a difference in one nucleotide (T450C) compared with the nucleotide sequence of CREBL1 that had already been registered in GenBank with accession number NM_00438. There is no change in the amino acid resulting from this difference. In the meantime, the termination codon was changed from TGA to TAA. It was confirmed that these differences were not due to PCR errors. The nucleotide sequence of CREBL1 DNA obtained in this example and the amino acid sequence encoded by the DNA are shown in SEQ ID NO: 15 and SEQ ID NO: 16, respectively. Animal cell expression plasmid for CREBL1 was prepared by digesting the cloned CREBL1 gene with BamHI and XhoI, and then integrated it into pCMV-Tag 3.

ATF6 gene was amplified by PCR using Mammary Gland QUICK-Clone cDNA (Clontech) as a template, ATF6Fn primer (SEQ ID NO: 27), ATF6Rn primer (SEQ ID NO: 28), and KOD-plus as DNA polymerase. Then, ATF6 gene was further amplified by PCR using the ATF6 gene thus amplified, as a template, using ATF6F1L primer (with EcoRV site immediately before ATG, SEQ ID NO: 29), ATF6R1L primer (with XhoI site immediately after the termination codon, SEQ ID NO: 30), and KOD-plus as DNA polymerase, and then cloned into pCR4Blunt-TOPO vector (Invitrogen). The nucleotide sequence was determined by the sequencer (SEQ ID NO: 17). It was found that the nucleotide sequence of ATF6 DNA thus obtained in this example had a difference in fifteen nucleotides compared with the nucleotide sequence that had already been registered in GenBank with accession number NM_007348. It was revealed that all of the fifteen different nucleotides coincided with the genome sequence: T105C (without amino acid change); T199A (with amino acid change from Leu to Met); A201 G (with amino acid change from Leu to Met); C270T (without amino acid change); A309G (without amino acid change); C433G (with amino acid change from Pro to Ala); T469C (with amino acid change from Ser to Pro); G1228A (with amino acid change from Gly to Ser); A1230C (with amino acid change from Gly to Ser); C1389T (without amino acid change); T1416C (without amino acid change); T1491A without amino acid change); T1538C (with amino acid change from Val to Ala); G1540C (with amino acid change from Val to Leu); and 1896A (without amino acid change). In the meantime, there is a description in SWISS-PROT about changes of all these amino acids in terms of conflict. The nucleotide sequence of ATF6 DNA obtained in this example and the amino acid sequence encoded by the DNA are shown in SEQ ID NO: 17 and SEQ ID NO: 18, respectively.

In order to integrate ATF6 gene into animal cell expression vector, ATF6 gene was amplified by PCR using the aforementioned ATF6 gene having been cloned into pCR4Blunt-TOPO vector as a template, using ATF6F2L primer (with BglII site immediately before ATG, SEQ ID NO: 31), ATF6R1L primer (SEQ ID NO: 30), and KOD-plus as DNA polymerase, and then cloned into pCR4Blunt-TOPO vector. The nucleotide sequence was determined by sequencer (SEQ ID NO: 19). ATF6 gene thus cloned was digested by BglII and XhoI to integrate into animal cell expression vector, pCMV-Tag3, digested with BamHI and XhoI.

### 4. Expression of CREBL1 and ATF6

HEK293T cells were cultured to 1.5 x 10⁶ cells /10 cm Dish. Next day, the expression plasmid of CREBL1 or ATF6 (10 µg/Dish) was transfected into HEK293T cells using FuGene 6 (Roche). Forty eight hours later, cells were washed with PBS, followed by adding 1 ml of lysis buffer B (50 mM Tris-HCl (pH 7.6), 150 mM NaCl, 1% Triton X-100, 1 % Nonidet P-40 (NP-40), Complete Mini-EDTA (ethylenediamine tetra-acetate) free), and left to stand on ice for 10 min. Then, cells were collected with a scraper, put into a 1.5 ml tube, subjected to sonication under ice-cooling (15 sec x 6 times), left to stand on ice for 20 min, suspended by pipetting, followed by subjecting centrifugation (15,000 rpm, 30 min, 4°C) to separate into a soluble fraction and an insoluble fraction. The soluble fractions were used as a test sample.

### 5. Determination of the activity of mature HtrA2 and mature HtrA2 (S306A)

Determination of the activity of mature HtrA2 and mature HtrA2 (S306A) was carried out with casein zymography and with a protease assay with casein sodium. Casein zymography was carried out according to the protocol (Invitrogen). More concretely, mature HtrA2 and mature HtrA2 (S306A) were mixed under non-reductive condition with equivalent volume of 2 x SDS sample buffer, left to stand at room temperature for 10 min, followed by separation with 4-16% zymogram (Blue casein) gel (Zymogram Gel, Invitrogen). After completion of electrophoresis, they were renatured with 2.5% Triton X-100, and then subjected to a degradation reaction of casein in the developing buffer at 37°C for overnight.

Meanwhile, a protease assay using casein sodium as an substrate was carried out in such that mature HtrA2 or mature HtrA2 (S306A) was mixed with casein sodium in a reaction buffer (150 mM NaCl, 50 mM Tris-HCl (pH 7.5)) so as to the concentration of the former might become 200 *µ*g/ml and the concentration of the latter might become 400 *µ*g/ml, and incubated at 37°C for reactions. A portion of the reaction solution was sampled at three hours and overnight after initiation of the reaction, mixed with equivalent volume of 2 x SDS sample buffer followed by boiling, and then subjected to SDS-PAGE. After that, the degradation of casein sodium was detected by Coomassie Brilliant Blue (CBB) staining.

As a result of the aforementioned investigation, the degradation of casein by mature HtrA2 was observed, but the degradation of casein was not observed with mature HtrA2 (S306A). From these findings, it has been confirmed that mature HtrA2 is of active type having protease activity, but mature HtrA2 (S306A) is of inactive type not exhibiting protease activity.

### <Method>

CREBL1 and ATF6 which were captured on the resin were used in the experiments in order to eliminate the influence of proteinaceous impurities contained in the soluble fractions prepared from the cells obtained by transfecting an expression plasmid of CREBL1 or ATF6. Three hundreds *µ*l of each soluble fraction of CREBL1 and ATF6 was dispensed to six tubes (Nos. 1 to 6), followed by adding 20 *µ*l of protein G sepharose 4 FF (Amersham) of 50% slurry that had been subjected to blocking with BSA, to each of tubes, subjected to overturned mixing at 4°C for 1 hour, and then subjected to centrifugation (10,000 rpm, 10 sec, 4°C) to collect the supernatant, and thus the pretreatment (pre-clean) was performed. The resultant supernatant was mixed with 1.7 *µ*l (2 *µ*g) of anti-Myc antibody (Invitrogen), subjected to overturned mixing at 4°C for 3 hours, followed by adding 20 *µ*l of protein G sepharose 4 FF that had been subjected to blocking with BSA, and then subjected to overturned mixing at 4°C overnight. Thus, CREBL1 and ATF6 were respectively captured on protein G sepharose 4 FF.

Subsequently, protein G sepharose 4 FF on which CREBL1 or ATF6 was captured was washed with 500 *µ*l of washing buffer (50 mM Tris-HCl (pH 7.5),150 mM NaCl, 0.01 % Triton X-100) five times, and 100 *µ*l of 50 mM Tris-HCl (pH 7.5),150 mM NaCl, 0.01 % Triton X-100 was added to the tubes Nos. 1 and 2, 100 *µ*l of mature HtrA2 solution (50 *µ*g/ml) was added to the tubes Nos. 3 and 4, and 100 *µ*l of mature HtrA2 (S306A) solution (50 *µ*g/ml) was added to the tubes Nos. 5 and 6, respectively. The tubes Nos. 1, 3, and 5 were subjected to a reaction at 37°C for four hours, the tubes Nos. 2, 4, 6 were subjected to a reaction overnight. After the reaction, the centrifugation was carried out to remove the supernatant. The precipitate was subjected to washing/centrifugation for three times with 500 *µ*l of washing buffer, followed by adding 80 *µ*l of 2 x SDS sample buffer (containing 0.1% β-mercaptoethanol) and boiling. Ten *µ*l of the sample thus obtained (80 *µ*l) was subjected to SDS-PAGE and transfered onto PVDF membrane to detect CREBL1 and ATF6 by western blotting. Anti-c-Myc antibody (9E 10) (Santa Cruz) was used as a primary antibody for detection, and horseradish peroxidase (HRP) conjugated anti-mouse IgG antibody (Cell Signaling) was used as a secondary antibody. Detection was carried out using ECL Western Blotting Detection System (Amersham).

### <Results>

It was observed that both CREBL1 and ATF6 were degraded by mature HtrA2 in vitro. As shown in Fig. 1-A, the band of CREBL1 was reduced significantly after the reaction of active HtrA2 with CREBL1 for overnight (O/N). Further, as shown in Fig. 1-B, the band of ATF6 was reduced significantly after the reaction of active HtrA2 with ATF6 for four hours (4h) or overnight (O/N). On the other hand, the degradation of CREBL1 and ATF6 by inactive HtrA2 (mature HtrA2 (S306A)) was not observed (Fig. 1-A and Fig. 1-B).

### Example 3

### <Intracellular protease assay>

Interaction of CREBL1 or ATF6 with HtrA2 was investigated by intracellular protease assay.

### <Materials and their preparations>

It has been reported that N-terminal four amino acid residues (AVPS) of mature HtrA2, which is a binding motif to IAPs (Inhibitor of apoptosis proteins) family protein acting for inhibiting cell death, bind to IAPs to inhibit the action, thereby to accelerate caspase dependent cell death. Since this action may be likely to give an influence upon the protease assay in a cell. Therefore, in order to inhibit the interaction of mature HtrA2 or mature HtrA2 (S306A) with IAPs, those (ΔAVPS) that lack the N-terminal four amino acid residues (AVPS) and those (AVPS → GVPS) with a substation of alanine among the four amino acid residues with glycine were prepared for each of mature HtrA2 and mature HtrA2 (S306A). Each of these various types of HtrA2 was prepared as C-termainal FLAG-tagged protein for use.

### 1. Preparation of various types of HtrA2

In order to prepare mature HtrA2 mutants, each gene of mature HtrA2 (ΔAVPS) and mature HtrA2 (GVPS) was amplified by PCR and cloned into pCR-BluntII-TOPO vector. The PCR was carried out using mature HtrA2 as a template, F1 and F2 primers (with SacI site immediately before ATG, SEQ ID NO: 32 and SEQ ID NO: 33, respectively) as the sense primer, HtrA2-RS primer (SEQ ID NO: 20) as the anti-sense primer, and Pfu turbo (Stratagene) as the DNA polymerase. The nucleotide sequence was determined by sequencer. Each of animal cell expression plasmids for mature HtrA2 (ΔAVPS) and mature HtrA2 (GVPS) was prepared by digesting each of the cloned mature HtrA2 (ΔAVPS) gene and the cloned mature HtrA2 (GVPS) gene by SacI and XhoI, and then integrated it into pCMV-Tag4. The nucleotide sequence of mature HtrA2 (ΔAVPS) DNA obtained in this example and the amino acid sequence encoded by the DNA are shown in SEQ ID NO: 7 and SEQ ID NO: 8, respectively. Besides, the nucleotide sequence of mature HtrA2 (GVPS) DNA and the amino acid sequence encoded by the DNA are shown in SEQ ID NO: 9 and SEQ ID NO: 10, respectively.

In order to prepare mature HtrA2 (S306A) mutants, with a same manner as mentioned above, each gene of mature HtrA2 (S306A, ΔAVPS) and mature HtrA2 (S306A, GVPS) was amplified by PCR using mature HtrA2 (S306A) as a template, and then cloned into pCR-BluntII-TOPO vector. Each of animal cell expression plasmids of mature HtrA2 (S306A, ΔAVPS) and mature HtrA2 (S306A, GVPS) was prepared by digesting each of the cloned mature HtrA2 (S306A, ΔAVPS) and the cloned mature HtrA2 (S306A, GVPS) by SacI and XhoI, and then integrated it into pCMV-Tag4. The nucleotide sequence of mature HtrA2 (S306A, ΔAVPS) DNA obtained in this example and the amino acid sequence encoded by the DNA are shown in SEQ ID NO: 11 and SEQ ID NO: 12, respectively. Besides, the nucleotide sequence of mature HtrA2 (S306A, GVPS) DNA and the amino acid sequence encoded by the DNA are shown in SEQ ID NO: 13 and SEQ ID NO: 14, respectively.

### 2. Determination of enzyme activity of various types of HtrA2

The enzyme activity of the various types of HtrA2 each of which was expressed in a cultured cell was determined by the protease assay using casein sodium as a substrate (see Example 2). As a result, it has been revealed that both mature HtrA2 (ΔAVPS) and mature HtrA2 (GVPS) are of active type having protease activity and that both mature HtrA2 (S306A, ΔAVPS) and mature HtrA2 (S306A, GVPS) are of inactive type without exhibiting protease activity.

### 3. Preparation of animal cell expression vectors for CREBL1 and ATF6

The vectors that were prepared by the same procedures used in Example 2 were used.

### <Method>

HEK293T cells were cultured to 1.5 x 10⁵ cells /10 cm Dish. Next day, the expression plasmid of CREBL1 or ATF6 was transfected into HEK293T cells together with the expression plasmid of various types of HtrA2 using FuGene 6 (Roche). Each of the expression plasmid of various HtrA2 at 1 µg/Dish was added in combination with CREBL1 expression plasmid or ATF6 expression plasmid at 1 *µ*g/Dish. Forty eight hours later, the cells in which the various types of HtrA2 were co-expressed with CREBL1 or ATF6 were added with 200 *µ*l of lysis buffer B and an equivalent volume of 2 x SDS sample buffer (containing 0.1 % β-mercaptoethanol), and then subjected to sonication followed by boiling to use as a test sample.

Expression of CREBL1 and ATF6 and the degradation of these proteins were detected by western blotting using 10 *µ*l of the test sample thus obtained. Anti-c-Myc (9E10) antibody was used as a primary antibody for detection, and horseradish peroxidase (HRP) conjugated anti-mouse IgG antibody (Cell Signaling) was used as a secondary antibody. Detection was carried out using ECL Western Blotting Detection System.

### <Results>

In the analysis using cells in which the active mutant (mature HtrA2 (ΔAVPS) or mature HtrA2 (GVPS)) was co-expressed with CREBL1, the band of CREBL1 was reduced significantly (upper panel of Fig 2-A). In the analysis using cells in which the active mutant was co-expressed with ATF6, the band of ATF6 was reduced significantly (upper panel of Fig 2-B). On the other hand, in the analysis using cells in which the inactive mutant (mature HtrA2 S306 (ΔAVPS) or mature HtrA2 S306 (GVPS)) was co-expressed with CREBL1, the reduction of the band of CREBL1 was not observed (upper panel of Fig. 2-A). Further, in the analysis using cells in which the inactive mutant was co-expressed with ATF6, the reduction of the band of ATF6 was not observed (upper panel of Fig. 2-B). The expression of each HtrA2 mutant in the cells was almost same among the cells (lower panel of Fig. 2-A and B).

As mentioned above, it has been revealed that both CREBL1 and ATF6 are degraded in the cells by active mature HtrA2 (ΔAVPS) or by active mature HtrA2 (GVPS). On the other hand, both CREBL1 and ATF6 are not degraded by inactive mature HtrA2 (S306A, ΔAVPS) or by inactive mature HtrA2 (S306A, GVPS).

### Example 4

### (Investigation of degradation pattern by HtrA2)

Degradation pattern of CREBL1 by HtrA2 was investigated. The investigation was performed by in vitro protease assay with HtrA2 using biotinylated CREBL1.

### <Materials and their preparations>

### 1. Mature HtrA2 and mature HtrA2 (S306A)

Mature HtrA2 and mature HtrA2 (S306A) were prepared by similar procedures as used in Example 2 for use.

### 2. animal cell expression plasmid for CREBL1

The animal cell expression plasmid for CREBL1 was prepared by digesting CREBL1, which had been cloned into pCR-BluntII-TOPO vector by similar procedures as used in Example 2, with BamHI and XhoI, and then integrating it into pcDNA3.1/His.

### 3. Preparation ofbiotinylated CREBL1 using in vitro translation reaction system

The preparation ofbiotinylated CREBL1 was performed by in vitro translation reaction system (T_{N}T® Transcription/Translation System; Promega) using rabbit reticulocyte lysate.
More concretely, first, 1.5 *µ*l of the animal cell expression plasmid for CREBL1 (1 *µ*g/*µ*l),1 *µ*l of 1 mM methionine, 1 *µ*l ofbiotinylated lysine tRNA (Promega), and 6.5 *µ*l of nuclease free water were added to 40 *µ*l of in vitro translation reaction solution (T_{N}T® Quick Master Mix) to obtain total volume of 50 *µ*l, and reacted for at 30°C for 1.5 hours. Following this, 12.5 *µ*l of 50 *µ*l reaction solution was sampled, followed by adding with 2 x SDS sample buffer and boiling, to detect the expression ofbiotinylated CREBL1 by western blotting. The detection was carried out using streptavidin HRP (Promega) and Transcend™ Chemiluminescent substrate (Transcend™ NonRadioactive Translation detection system; Promega). As a result, biotinylation of CREBL1 was confirmed in sample.

### <Methods>

The reaction solution was used as the sample for protease assay. 12.5 *µ*l each of the reaction solution was dispensed to three tubes (Nos. 2 to 4). 25 *µ*l of 50 mM Tris-HCl (pH 7.5), 150 mM NaCl, 0.01 % Triton X-100 was added as the control to tube No.2,25 *µ*l of 200 *µ*g/ml mature HtrA2 solution was added to tube No.3, and 25 *µ*l of 200µg/ml mature HtrA2 (S306A) solution was added to tube No.4, respectively, and mixed. The solution in each tube (Nos. 2 to 4) was further divided into two tubes so as to the tubes include an equivalent volume of solution, while one tube was subjected to reaction at 37°C for four hours and the other tube was subjected to reaction at 37°C overnight. After the reaction, 2 x SDS sample buffer was added to each of tubes, and subsequently subjected to boiling, to detect the degradation pattern ofbiotinylated CREBL1 by western blotting.

The detection of the degradation was carried out using streptavidin HRP and Transcend™ Chemiluminescent substrate while biotinylated lysine residue in CREBL1 was used as the index.

Further, in order to determine the degradation site, the membrane used for western blotting was subjected to treatment for removing the streptavidin HRP, and then used for detecting the degradation pattern of biotinylated CREBL1 using an antibody against the tag ligated to the N-terminal of CREBL1. Anti-Xpress antibody (Invitrogen) was used as a primary antibody, and HRP labeled anti-mouse IgG antibody (Cell Signaling) was used as a secondary antibody. The detection of degradation was carried out using ECL Western Blotting Detection Reagents (Amersham).

### <Results>

Results of the study for the degradation pattern of CREBL1 by mature HtrA2 using biotinylated lysine residue in CREBL1 as an index are shown in Fig. 3. Similarly, results of the study for the degradation pattern of CREBL1 using a tag ligated to the N-terminal of CREBL1 as an index are shown in Fig. 4.

In the study for the degradation pattern using biotinylated lysine residue as an index, remarkable reduction of the band of biotinylated CREBL1 was observed, and further, the band that was supposed to be a degradation product of CREBL1 was detected around 50 kDa (Fig. 3). However, in the study for the degradation pattern using the anti-tag antibody, the band that was supposed to be a degradation product of CREBL1 which was detected around 50 kDa in Fig. 3 could not be detected. Moreover, any band indicating the degradation product of CREBL1 with different size was not detected (Fig. 4). From this findings, it is believed that CREBL1 was degraded by being cleaved by HtrA2 at several sites.

As described above, the degradation of CREBL1 by HtrA2 was detected by detecting a labeled chemical that was used for labeling the inside of CREBL1. From this finding, it has been revealed that the degradation of CREBL1 by HtrA2 identified by detection of the tag ligated to the N-terminal of CREBL1 (Examples 2 and 3) is not an apparent degradation due to the cleavage of the tag, but is a degradation resulting from the cleavage of peptide bond of CREBL1 caused by the effect of HtrA2 on CREBL1.

### Example 5

### (In-silico search for proteins having a function to interact with HtrA2)

The prediction of proteins that have a function to interact with HtrA2 was conducted according to the method described in the pamphlet of International Publication No. WO 01/67299. Concretely, the amino acid sequence of HtrA2 was decomposed into oligopeptides having a predetermined length in order to search in a database for proteins having the amino acid sequence of each of the oligopeptides, or having homologous amino acid sequences to these amino acid sequences. Then, local alignment was conducted between the proteins obtained and HtrA2 to identify proteins having a high local alignment score that might be capable of interacting with HtrA2.

As a result of analysis, HNF-4α was identified as a protein being predicted to have a function to interact with HtrA2.

### Example 6

### (In vitro assay)

In order to demonstrate the interaction of HNF-4α with HtrA2, in vitro protease assay was performed.

### <Materials and their preparations>

In this Example, mature HtrA2 (SEQ ID NO: 4) and mature HtrA2 (S306A) (SEQ ID NO: 6) to each of which histidine (His)-tag was ligated at the C-terminal were used as active HtrA2 and inactive HtrA2, respectively. Further, HNF-4α (SEQ ID NO: 35) to which (6 x His) -Xpress-tag was ligated at the N-terminal was used as HNF-4α

### Mature HtrA2 and mature HtrA2 (S306A)

Mature HtrA2 and mature HtrA2 (S306A) were prepared by similar procedures as used in Example 2.

### 2. Preparation of HNF-4α expression plasmid

HNF-4α gene was amplified by PCR using Human Brain polyA⁺RNA as a template. A nucleotide substitution that may be a result of PCR error was corrected with Quick Change Site-Directed Mutagenesis kit (Stratagene). After that, HNF-4α gene was integrated into animal cell expression plasmid pcDNA3.1/His (Invitrogen) that gives a (6 x His)-Xpress-tagged N-terminal, to construct HNF-4α expression plasmid (HNF-4α/pcDNA3.1/His). The deduced amino acid sequence encoded by the cloned HNF-4α DNA was identical with the amino acid sequence of HNF-4α disclosed in Swiss-Prot database with the accession number P41235 (registered gene name is HNF4A). The nucleotide sequence of HNF-4α DNA obtained in this Example and the amino acid sequence encoded by the DNA are shown in SEQ ID NO: 34 and SEQ ID NO: 35, respectively.

Using the HNF-4α expression plasmid, recombination of HNF-4α DNA to animal cell expression plasmid pCMV-Tag2 (Stratagene) that gives a FLAG-tagged N-terminal at EcoRI site was carried out to construct the HNF-4α expression plasmid (HNF-4α/pCMV-Tag2).

### 3. Preparation of HNF-4α

HEK293T cells were cultured to 1.5 x 10⁶ cells /10 cm Dish. Next day, the expression plasmid of HNF-4α (10 µg/Dish) was transfected into HEK293T cells using FuGene 6 (Roche). Forty eight hours later, cells were washed with PBS, followed by adding 1 ml of lysis buffer B (50 mM Tris-HC1 (pH 7.6), 150 mM NaCI, 1% Triton X-100, 1% Nonidet P-40 (NP-40), Complete Mini-EDTA free), and left to stand on ice for 10 min. Then, cells were collected with a scraper, put into a 1.5 ml tube, subjected to sonication under ice-cooling (15 sec x 6 times), left to stand on ice for 20 min, suspended by pipetting, followed by subjecting centrifugation (15,000 rpm, 30 min, 4°C) to separate into a soluble fraction and an insoluble fraction. The soluble fractions were used as a test sample. (hereinafter to be referred to as HNF-4α soluble fraction).

### <Methods>

HNF-4α captured on the resin by immunoprecipitation was used in the experiments in order to eliminate the influence of proteinaceous impurities contained in the HNF-4α soluble fraction. More specifically, 300 *µ*l of HNF-4α soluble fraction was dispensed to six tubes (Nos. 1 to 6), followed by adding 20 *µ*l of protein G sepharose 4 FF (Amersham) of 50% slurry that had been subjected to blocking with BSA, to each of tubes, subjected to overturned mixing at 4°C for 1 hour, and then subjected to centrifugation (10,000 rpm, 10 sec, 4°C) to collect the supernatant, and thus the pretreatment (pre-clean) was performed. The resultant supernatant was mixed with 0.4 *µ*l (2 *µ*g) of anti-FLAG antibody (Sigma) by overturned mixing at 4°C for 3 hours, followed by adding 20 *µ*l of protein G sepharose 4 FF that had been subjected to blocking with BSA, and then subjected to overturned mixing at 4°C overnight. Thus, HNF-4α was captured on protein G sepharose 4 FF.

Subsequently, protein G sepharose 4 FF on which HNF-4α was captured was washed with 500 *µ*l of washing buffer (50 mM Tris-HCl (pH 7.5), 150 mM NaCl, 0.01% Triton X-100) five times, and 100 *µ*l of 50 mM Tris-HCl (pH 7.5),150 mM NaCl, 0.01% Triton X-100 was added to tubes Nos. 1 and 2,100 *µ*l of mature HtrA2 solution (50 *µ*g/ml) was added to tubes Nos. 3 and 4, and 100 *µ*l of mature HtrA2 (S306A) solution (50 *µ*g/ml) was added to tubes Nos. 5 and 6, respectively. Tubes Nos. 1, 3 and 5 were subjected to a reaction at 37°C for four hours, tubes Nos. 2, 4, 6 were subjected to a reaction overnight. After the reaction, the centrifugation was carried out to remove the supernatant. The precipitate was subjected to washing/centrifugation for three times with 500 *µ*l of washing buffer, followed by adding 80 *µ*l of 2 x SDS sample buffer (containing 0.1 % β-mercaptoethanol) and boiling. Ten µ*l* of the sample thus obtained (80 *µ*l) was subjected to SDS-PAGE and transfered onto PVDF membrane to detect HNF-4α by western blotting. Anti-FLAG antibody (Sigma) was used as a primary antibody for detection, and horseradish peroxidase (HRP) labeled anti-mouse IgG antibody (Cell Signaling) was used as a secondary antibody. Detection was carried out using ECL Western Blotting Detection System (Amersham).

### <Results>

It was observed that HNF-4α was degraded by mature HtrA2 in vitro. As shown in Fig. 5, the band of HNF-4α was reduced significantly after the reaction of active HtrA2 with HNF-4α for overnight (O/N). On the other hand, the degradation of HNF-4α by inactive HtrA2 (mature HtrA2 (S306A)) was not observed (Fig. 5).

### Example 7

### (Intracellular protease assay)

Interaction of HNF-4awith HtrA2 was investigated by intracellular protease assay.

### <Materials and their preparations>

HNF-4α animal cell expression plasmid (HNF-4α/pCMV-Tag2) prepared in Example 6 was used as an HNF-4α expression plasmid. Further, as an HtrA2 expression plasmid, mature HtrA2 (ΔAVPS) expression plasmid, mature HtrA2 (GVPS) expression plasmid, mature HtrA2 (S306A, ΔAVPS) expression plasmid, and mature HtrA2 (S306A, ΔAVPS) expression plasmid were used, all of which were prepared in Example 3.

### <Methods>

HEK293T cells were cultured to 1.5 x 10⁵ cells /6 cm Dish. Next day, the expression plasmid of HNF-4α was transfected into HEK293T cells together with the expression plasmid of various types of HtrA2 using FuGene 6 (Roche). Each of the expression plasmid of various HtrA2 at 1 µg/Dish was added in combination with HNF-4a expression plasmid at 1 µg/Dish. Forty eight hours later, the cells in which the various types of HtrA2 were co-expressed with HNF-4α were added with 200 *µ**l of lysis buffer B and an equivalent volume of 2 x SDS sample buffer (containing 0.1% β-mercaptoethanol), and then subjected to sonication followed by boiling to use as a test sample.

Expression of HNF-4α and the degradation of HNF-4α were detected by western blotting using 10 *µ*l of the test sample thus obtained. Anti-FLAG antibody (Sigma) was used as a primary antibody for detection, and horseradish peroxidase (HRP) labeled anti-mouse IgG antibody (Cell Signaling) was used as a secondary antibody. Detection was carried out using ECL Western Blotting Detection System.

### <Results>

In the analysis using cells in which the active mutant (mature HtrA2 (ΔAVPS) or mature HtrA2 (GVPS)) was co-expressed with HNF-4α, the band of HNF-4α was reduced significantly (Fig. 6). On the other hand, in the analysis using cells in which the inactive mutant (mature HtrA2 S306 (ΔAVPS) or mature HtrA2 S306 (GVPS)) was co-expressed with HNF-4α, the reduction of the band of HNF-4α was not observed (Fig. 6).

As mentioned above, it has been revealed that HNF-4α is degraded in the cells by active mature HtrA2 (ΔAVPS) or by active mature HtrA2 (GVPS). On the other hand, HNF-4α was not degraded by inactive type mature HtrA2 (S306A, ΔAVPS) or inactive type mature HtrA2 (S306A, GVPS).

### INDUSTRIAL APPLICABILITY

The present invention can be utilized in prevention and/or treatment of diabetes attributable to the degradation by HtrA2 of at least one of CREBL1, ATF6, and HNF-4α, and in inhibition of cell death (for example, pancreatic *β* cell death) attributable to the degradation by HtrA2 of CREBL1 and/or ATF6. Thus, the present invention is extremely useful in the pharmaceutical fields.

### SEQUENCE LISTING FREE TEXT

SEQ ID NO: 1: DNA encoding HtrA2 precursor protein
SEQ ID NO: 2: HtrA2 precursor protein
SEQ ID NO: 3: DNA encoding mature HtrA2
SEQ ID NO: 4: mature HtrA2
SEQ ID NO: 5: Polynucleotide consisting of the same nucleotide sequence of SEQ ID NO: 3, wherein the nucleotide of position 520 is g; DNA encoding mature HtrA2 (S306A)
SEQ ID NO: 6: Polypeptide consisting of the same amino acid sequence of SEQ ID NO: 4, wherein the 174th amino acid residue is substituted by Ala; mature HtrA2 (S306A)
SEQ ID NO: 7: Polynucleotide consisting of the same nucleotide sequence of SEQ ID NO: 3, wherein the nucleotides of position 4-15 are deleted; DNA encoding mature HtrA2 (delta AVPS)
SEQ ID NO: 8: Polypeptide consisting of the same amino acid sequence of SEQ ID NO: 4, wherein the amino acid residues from the 2nd to the 5th are deleted; mature HtrA2 (delta AVPS)
SEQ ID NO: 9: Polynucleotide consisting of the same nucleotide sequence of SEQ ID NO: 3, wherein the nucleotide of position 5 is g; DNA encoding mature HtrA2 (GVPS)
SEQ ID NO: 10: Polypeptide consisting of the same amino acid sequence of SEQ ID NO: 4, wherein the 2nd amino acid residue is substituted by Gly; mature HtrA2 (GVPS)
SEQ ID NO: 11: Polynucleotide consisting of the same nucleotide sequence of SEQ ID NO: 5, wherein the nucleotides of position 4-15 are deleted; DNA encoding mature HtrA2 (S306A, delta AVPS)
SEQ ID NO: 12: Polypeptide consisting of the same amino acid sequence of SEQ ID NO: 6, wherein the amino acid residues from the 2nd to the 5th are deleted; mature HtrA2 (S306A, delta AVPS)
SEQ ID NO: 13: Polynucleotide consisting of the same nucleotide sequence of SEQ ID NO: 5, wherein the nucleotide of position 5 is g; DNA encoding mature HtrA2 (S306A, GVPS)
SEQ ID NO: 14: Polypeptide consisting of the same amino acid sequence of SEQ ID NO: 6, wherein the 2nd amino acid residue is substituted by Gly; mature HtrA2 (S306A, GVPS)
SEQ ID NO: 15: DNA encoding CREBL1
SEQ ID NO: 16: CREBL1
SEQ ID NO: 17: DNA encoding ATF6
SEQ ID NO: 18: ATF6
SEQ ID NO: 19: Designed polynucleotide based on the nucleotide sequence of SEQ ID NO: 3 for use as a primer to obtain mature HtrA2 DNA
SEQ ID NO: 20: Designed polynucleotide based on the nucleotide sequence of SEQ ID NO: 3 for use as a primer to obtain mature HtrA2 DNA
SEQ ID NO: 21: Designed polynucleotide based on the nucleotide sequence of SEQ ID NO: 3 for use as a primer to obtain mature HtrA2 (S306A) DNA
SEQ ID NO: 22: Designed polynucleotide based on the nucleotide sequence of SEQ ID NO: 3 for use as a primer to obtain mature HtrA2 (S306A) DNA
SEQ ID NO: 23: Designed polynucleotide based on the nucleotide sequence of SEQ ID NO: 15 for use as a primer to obtain CREBL1 DNA
SEQ ID NO: 24: Designed polynucleotide based on the nucleotide sequence of SEQ ID NO: 15 for use as a primer to obtain CREBL1 DNA
SEQ ID NO: 25: Designed polynucleotide based on the nucleotide sequence of SEQ ID NO: 15 for use as a primer to obtain CREBL1 DNA
SEQ ID NO: 26: Designed polynucleotide based on the nucleotide sequence of SEQ ID NO: 15 for use as a primer to obtain CREBL1 DNA
SEQ ID NO: 27: Designed polynucleotide based on the nucleotide sequence of SEQ ID NO: 17 for use as a primer to obtain ATF6 DNA
SEQ ID NO: 28: Designed polynucleotide based on the nucleotide sequence of SEQ ID NO: 17 for use as a primer to obtain ATF6 DNA
SEQ ID NO: 29: Designed polynucleotide based on the nucleotide sequence of SEQ ID NO: 17 for use as a primer to obtain ATF6 DNA
SEQ ID NO: 30: Designed polynucleotide based on the nucleotide sequence of SEQ ID NO: 17 for use as a primer to obtain ATF6 DNA
SEQ ID NO: 31: Designed polynucleotide based on the nucleotide sequence of SEQ ID NO: 17 for use as a primer to obtain ATF6 DNA
SEQ ID NO: 32: Designed polynucleotide based on the nucleotide sequence of SEQ ID NO: 3 for use as a primer to obtain mature HtrA2 (delta AVPS) DNA
SEQ ID NO: 33: Designed polynucleotide based on the nucleotide sequence of SEQ ID NO: 3 for use as a primer to obtain mature HtrA2 (GVPS) DNA
SEQ ID NO: 34: DNA encoding HNF-4alpha
SEQ ill NO: 35: HNF-4α

## Claims

1. A method for inhibiting the degradation of at least one of CREBL1 (cAMP responsive element binding protein-like 1), ATF6 (activating transcription factor 6), and HNF-4α (hepatocyte nuclear factor-4α), comprising inhibiting the function of HtrA2 (high temperature requirement protein A2).

2. The method for inhibiting the degradation of at least one of CREBL1, ATF6, and HNF-4α according to claim 1, in which to inhibit the function of HtrA2 is to inhibit the cleavage by HtrA2 of at least one of CREBL1, ATF6, and HNF-4α, wherein to inhibit the cleavage by HtrA2 of CREBL1 brings inhibition of the degradation of CREBL1, to inhibit the cleavage by HtrA2 of ATF6 brings inhibition of the degradation of ATF6, and to inhibit the cleavage by HtrA2 of HNF-4α brings inhibition of the degradation ofHNF-4α.

3. The method for inhibiting the degradation of at least one of CREBL1, ATF6, and HNF-4α according to claim 1, in which to inhibit the function ofHtrA2 is to inhibit the interaction of the HtrA2 with at least one of CREBL1, ATF6, and HNF-4α, wherein to inhibit the interaction of the HtrA2 with CREBL1 brings inhibition of the degradation of CREBL1, to inhibit the interaction of the HtrA2 with ATF6 brings inhibition of the degradation of ATF6, and to inhibit the interaction of the HtrA2 with HNF-4α brings inhibition of the degradation of HNF-4α.

4. A method for preventing and/or treating diabetes, comprising inhibiting the degradation by HtrA2 of at least one of CREBL1, ATF6, and HNF-4α.

5. A method for preventing and/or treating diabetes, comprising using the method for inhibiting the degradation of at least one of CREBL1, ATF6, and HNF-4α according to any one of claims 1 to 3.

6. A method for preventing and/or treating diabetes, comprising using one or more compounds that inhibit the degradation by HtrA2 of at least one of CREBL1, ATF6, and HNF-4α

7. An agent for preventing and/or treating diabetes, comprising one or more compounds that inhibit the degradation by HtrA2 of at least one of CREBL1, ATF6, and HNF-4α

8. A method of identifying a compound that inhibits the degradation by HtrA2 of at least one of CREBL1, ATF6, and HNF-4α, comprising contacting at least one of CREBL1, ATF6, and HNF-4α and/or HtrA2 with a compound (a test compound) under conditions that allow the degradation by HtrA2 of at least one of CREBL1, ATF6, and HNF-4α, introducing a system using a signal and/or a marker capable of detecting at least one of CREBL1, ATF6, and HNF-4α; detecting the presence or absence and/or change of the signal and/or the marker; and determining whether the test compound inhibits the degradation of at least one of CREBL1, ATF6, and HNF-4α

9. A method of identifying a compound that inhibits the degradation by HtrA2 of at least one of CREBL1, ATF6, and HNF-4α, comprising contacting at least one of CREBL1, ATF6, and HNF-4α and/or HtrA2 with a compound (a test compound) under conditions that allow the degradation by HtrA2 of at least one of CREBL1, ATF6, and HNF-4α; detecting the presence or absence of at least one of CREBL1, ATF6, and HNF-4α, and/or measuring the change of the amount thereof; or detecting the presence or absence of the degradation product of at least one of CREBL1, ATF6, and HNF-4α, and/or measuring the change of the amount thereof; and determining whether the test compound inhibits the degradation of at least one of CREBL1, ATF6, and HNF-4α

10. The method according to claim 8 or 9, wherein the method of identifying a compound that inhibits the degradation by HtrA2 of at least one of CREBL1, ATF6, and HNF-4α is a method of identifying a compound that is an active ingredient in an agent for preventing and/or treating diabetes.

11. A method for inhibiting cell death, comprising inhibiting the degradation by HtrA2 of CREBL1 and/or ATF6.

12. A method for inhibiting cell death, comprising using one or more compounds that inhibit the degradation by HtrA2 of CREBL1 and/or ATF6.

13. The method for inhibiting cell death according to claim 11 or 12, in which the cell death is cell death of a pancreatic *β* cell.

14. An agent for inhibiting cell death, comprising one or more compounds that inhibit the degradation by HtrA2 of CREBL1 and/or ATF6.

15. The agent for inhibiting cell death according to claim 14, wherein the cell death is cell death of a pancreatic *β* cell.

16. A method for preventing and/or treating diabetes, comprising using the method for inhibiting cell death according to any one of claims 11 to 13.

17. A method for preventing and/or treating type 2 diabetes, comprising inhibiting the degradation by HtrA2 of HNF-4α.

18. An agent for preventing and/or treating type 2 diabetes, comprising one or more compounds that inhibit the degradation by HtrA2 ofHNF-4α

19. A reagent kit, comprising at least one selected from the group consisting of HtrA2, a polynucleotide encoding HtrA2, and a vector containing the polynucleotide encoding HtrA2; and at least one selected from the group consisting of CREBL1, ATF6, HNF-4α a polynucleotide encoding CREBL1 or ATF6 or HNF-4α, and a vector containing the polynucleotide encoding CREBL1 or ATF6 or HNF-4α.
